(19) Europäisches Patentamt / European Patent Office / Office européen des brevets

(11) **EP 3 984 485 B1**

(12) **EUROPEAN PATENT SPECIFICATION**

(45) Date of publication and mention
of the grant of the patent:
**23.10.2024   Bulletin 2024/43**

(21) Application number: **21202120.8**

(22) Date of filing: **12.10.2021**

(51) International Patent Classification (IPC):
*A61B 34/10* *(2016.01)*       *A61B 34/20* *(2016.01)*
*A61B 17/00* *(2006.01)*       *A61B 90/00* *(2016.01)*

(52) Cooperative Patent Classification (CPC):
**A61B 34/20; A61B 34/10;** A61B 2017/00119;
A61B 2017/00725; A61B 2034/102;
A61B 2034/105; A61B 2034/107; A61B 2034/2051;
A61B 2034/2055; A61B 2034/2063;
A61B 2034/207; A61B 2090/3937;
A61B 2090/3945; A61B 2090/397;
A61B 2090/3979;                              (Cont.)

(54) **TECHNIQUE FOR DETERMINING A RADIUS OF A SPHERICALLY SHAPED SURFACE PORTION OF AN INSTRUMENT TIP**

VERFAHREN ZUR BESTIMMUNG DES RADIUS EINES KUGELFÖRMIGEN OBERFLÄCHENABSCHNITTS EINER INSTRUMENTENSPITZE

TECHNIQUE DE DÉTERMINATION DE RAYON D'UNE PARTIE DE SURFACE DE FORME SPHÉRIQUE D'UNE EXTRÉMITÉ D'INSTRUMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **15.10.2020   EP 20201992**

(43) Date of publication of application:
**20.04.2022   Bulletin 2022/16**

(73) Proprietor: **Stryker European Operations Limited Carrigtwohill, Co. Cork T45 HX08 (IE)**

(72) Inventors:
• **GHANAM, Fadi**
  **79227 Schallstadt (DE)**
• **GUTH, Yannik**
  **79336 Herbolzheim (DE)**

(74) Representative: **Röthinger, Rainer**
**Wuesthoff & Wuesthoff**
**Patentanwälte und Rechtsanwalt PartG mbB**
**Schweigerstrasse 2**
**81541 München (DE)**

(56) References cited:
**US-A1- 2019 000 571       US-A1- 2019 142 527**
**US-A1- 2020 046 454       US-A1- 2020 405 400**

EP 3 984 485 B1

(52) Cooperative Patent Classification (CPC): (Cont.)
A61B 2090/3983

**Description**

**Technical Field**

The present disclosure generally relates to instrument tracking, for example in the context of surgical navigation. In particular, a method, a computer program product, and a device for determining a radius of a spherically shaped instrument tip are presented.

**Background**

Surgical navigation systems are typically configured to track a surgical instrument operated by the surgeon relative to a patient or other object. Based on the tracked surgical instrument, the surgical navigation system provides visual or acoustic instructions to guide the surgeon when operating the surgical instrument.

A common procedure for surgical navigation is to track both the surgical instrument and the patient. The surgical navigation system can thus display image data of the patient (e.g., a computer tomography scan) relative to a visual representation of the surgical instrument or a part thereof. In such a visual representation, a tip of the surgical instrument is commonly represented in form of a point or icon.

Tissue manipulation such as cutting, milling or drilling is largely affected by a size of the instrument tip. The following two examples illustrate that visually representing the instrument tip in the form of a point can impair the accuracy of the guidance provided to the surgeon.

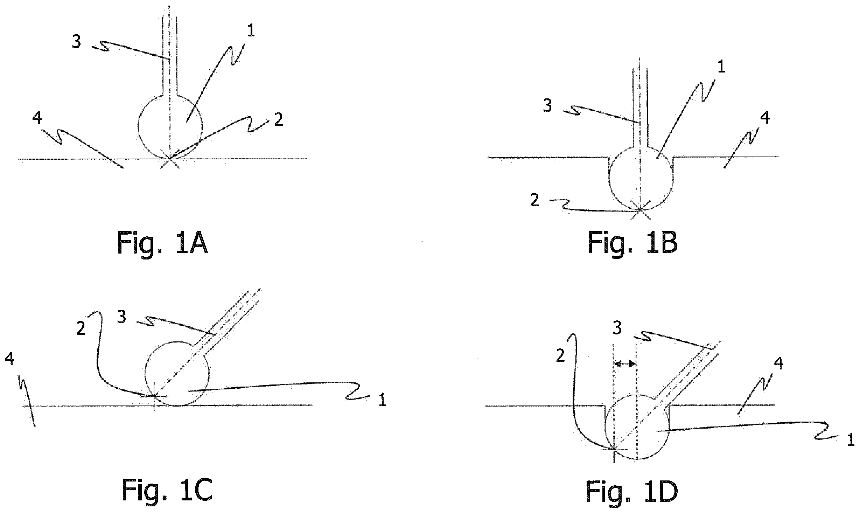

Fig. 1A shows a spherically shaped instrument tip 1 (e.g., a burr) having a distal end point 2. An instrument axis 3 extending through the end point 2 is arranged perpendicularly to a surface of tissue 4 (e.g., bone). Fig. 1B shows an opening that has been cut by the instrument tip 1 into the tissue 4 in a direction parallel to the instrument axis 3. Since the instrument axis 3 extends parallel to the opening cut into the tissue 4, the end point 2 is positioned at a bottom of the opening.

When visually representing a calculated location of the end point 2 relative to the tissue 4 on a display, the location of the visualized end point 2 properly coincides with the bottom of the opening. As such, the surgeon is correctly informed about, for example, an extension and a depth of the opening.

Fig. 1C shows the same instrument tip 1 as shown in Fig. 1A, but with the instrument axis 3 oriented at a non-perpendicular angle relative to the surface of the tissue 4. Therefore, when cutting an opening into the tissue 4 perpendicular to the surface of the tissue 4, the instrument axis 3 does not extend parallel to the opening. Fig. 1D shows that cutting an opening into the tissue 4 in the scenario of Fig. 1C results in the path of the end point 2 to be arranged offset to the cutting path of the opening. When a surgical navigation system uses the end point 2 as reference for the cutting path, the calculated cutting path will by offset relative to the actual cutting path. As such, the extension of the cutting path cannot be properly visualized. Moreover, the calculated location of the end point 2 is no longer indicative of a depth of the opening.

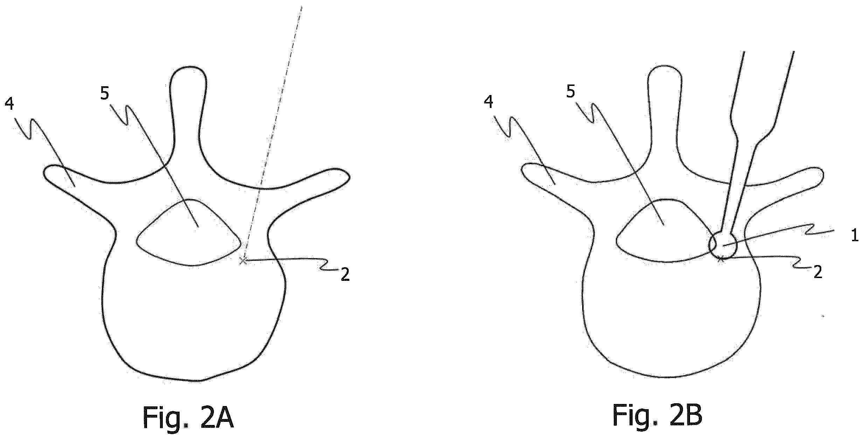

Fig. 2A illustrates an exemplary visual guidance that may be provided to the surgeon on a display. The visual guidance includes displaying a visual representation of a spherically shaped instrument tip in the form of an end point 2 relative to image data of tissue 4. The image data comprises a non-target area 5 that must not be affected by the surgical instrument. The visual guidance shown in Fig. 2A visualizes the end point 2 outside the non-target area 5, seemingly indicating that the instrument is not cutting into the non-target area 5. Fig. 2B shows the actual tissue 4 with the actual instrument tip 1. Since the end point 2 shown in Fig. 2A does not account for the radius of the instrument tip 1, the visual guidance of Fig. 2A does not indicate that the instrument tip 1 is in fact cutting into the non-target area 5.

As described in the two scenarios above, a visual representation in form of a point does not take the form, size and orientation of the instrument tip into account. Such an incomplete visualization can negatively affect the accuracy of the guiding instructions provided by the surgical navigation system.

The surgical instrument described with reference to Figs. 1A to 1D and Figs. 2A and 2B has an instrument tip with a spherically shaped surface portion that defines a radius, which may be unknown or require verification. The radius of the instrument tip can differ due to the availability of a set of instrument tips from which one may be selected by the surgeon depending on the prevailing surgical situation.

In order to provide the surgeon with accurate guiding information in the scenarios discussed above, and in other scenarios, the surgical navigation system requires precise knowledge of the radius of the spherically shaped surface portion. In some cases, the instruments are provided with labels that indicate the radius of their tips. However, relying on the surgeon to properly input the selected instrument or the associated tip radius via a keyboard or graphical user interface has turned out to be error-prone and tedious during on-going surgery. Moreover, the use of labels does not take into account a radius change caused by wear or other effects. As a consequence, relying on the surgeon to properly identify the radius of a selected instrument tip is error prone, time-inefficient and potentially unsafe for the patient.

Document US 2020/405400 A1 discloses surgical instrument calibration methods, systems, and devices that allow a virtual representation of a surgical instrument to be modified to adjust for any variations in a distal tip of a surgical instrument. An instrument calibration system is disclosed that can have a surgical instrument, a calibration instrument, and a monitoring system. The surgical instrument can have a distal tip and an orientation element thereon, and the calibration instrument can have a pivot point thereon and a calibration reference element attached thereto. The monitoring system can be configured to record movement of the surgical instrument with respect to the calibration instrument when the tip of the surgical instrument is inserted into the pivot point of the calibration instrument, and to calculate a deviation of the tip of the surgical instrument from a predefined ideal tip based on the recorded movement.

Document US 2020/046454 A1 discloses a registration and identification tool for an instrument. The instrument comprises a body, a marker member which is optically detectable and provided on the body, and a recess in the body which extends from an outer surface of the body into the inside of the body, thereby defining an extension direction of the recess. The recess has a shape such that a lateral extension of the recess decreases in the direction from the outer surface of the body towards the inside of the body. A method for registration and identification of an instrument comprises placing a surgical tool of the surgical instrument into the recess of the tool, pivoting the surgical instrument relative to the marker member while the surgical tool is placed inside the recess, performing a detecting process of the relative movement of the surgical instrument, and identifying geometrical characteristics of the surgical tool and/or registering the relative position of the surgical tool to the remainder of the surgical instrument using the results of the detection process.

## Summary

There is a need for a technique that solves one or more of the aforementioned or other problems.

According to one aspect, a computer-implemented method for determining a radius of a spherically shaped surface portion of a tip of an instrument is provided. A calibration device is provided that comprises a flat calibration surface and a calibration structure, wherein the calibration structure defines an opening angle and a real or imaginary first reference point relative to which the opening angle is defined. The calibration device comprises a first tracker trackable by a tracking system and arranged in a first predetermined relationship relative to the first reference point and in a second predetermined relationship relative to the calibration surface. The instrument comprises a second tracker trackable by the tracking system. The method comprises multiple steps performed by a computer system. The method comprises determining a first pose of the first tracker and a second pose of the second tracker while the spherically shaped surface portion is in abutment with the calibration structure. The method further comprises determining a reference position of the first reference point relative to the second tracker when the first tracker is in the first pose and the second tracker is in the second pose based on the first pose, the second pose, and the first predetermined relationship. The method comprises determining a third pose of the first tracker and a fourth pose of the second tracker while the spherically shaped surface portion is in abutment with the calibration surface. The method further comprises determining the radius of the spherically shaped surface portion based on the third pose, the second predetermined relationship, the reference position, the fourth pose, and the opening angle.

Determining the radius of the spherically shaped surface portion may comprise determining a second reference point based on the fourth pose and the reference position, determining a reference distance defining a distance between the second reference point and the calibration surface based on the second reference point, the third pose, and the second predetermined relationship, and determining the radius based on the reference distance and a trigonometric function of the opening angle.

The second pose may define a first calibration orientation of the second tracker and the fourth pose may define a second calibration orientation of the second tracker. A reorientation angle may define an angular difference between the first calibration orientation and the second calibration orientation, and a surface angle may define an angular difference between a normal vector of the calibration surface and a bisector of the opening angle. In such a scenario, the method may further comprise determining a pivot angle that defines an angle based on the reorientation angle and the surface angle. The pivot angle may be determined as a polar angular component in regard to the surface normal of a sum of the reorientation angle and the surface angle.

Determining the radius of the spherically shaped surface portion may further be based on the pivot angle.

The method may comprise determining a position of a centre point of the spherically shaped surface portion relative to the second tracker based on the third pose, the second predetermined relationship, the reference position, the fourth pose, the opening angle, and the radius. The method may comprise determining a second reference point based on the fourth pose and the reference position. The method may comprise determining a reference axis through the second reference point and perpendicular to the calibration surface. The method may further comprise determining a position of a centre point of the spherically shaped surface portion relative to the second tracker along the reference axis at a distance of the radius from the reference surface in a direction away from the calibration device.

The method may further comprise rotating the reference axis around the second reference point by the pivot angle before determining the position of the centre point along the reference axis.

According to a second aspect, a computer-implemented method for determining a radius of a spherically shaped

# EP 3 984 485 B1

surface portion of a tip of an instrument is provided. The spherically shaped surface portion has a centre point. A calibration device is provided that comprises a calibration structure defining an opening angle and that is configured to cooperate with the spherically shaped surface portion so as to guide a tilting movement of the instrument tip around the centre point. The calibration device comprises a first tracker trackable by a tracking system and arranged in a predetermined relationship relative to the calibration structure. The instrument comprises a second tracker trackable by the tracking system. The method is performed by a computer system and comprises determining a first pose of the first tracker. The method further comprises determining positional data of the second tracker for different tilting positions of the instrument while the spherically shaped surface portion is in abutment with the calibration structure. The method further comprises determining a position of the centre point relative to the second tracker based on the positional data. The method also comprises determining the radius of the spherically shaped surface portion based on the position of the centre point, the first pose, the predetermined relationship, and the opening angle.

The method of the second aspect may comprise determining the radius based on a centre distance between the position of the centre point, a real or imaginary point relative to which the opening angle is defined and a trigonometric function of the opening angle. As an example, determining the radius may be based on the sinus of half the opening angle. Evidently, the sinus can be replaced by other trigonometric functions as generally known in the art.

The following observations pertain to both the method of the first aspect and the method of the second aspect.

The method may comprise tracking a third tracker of an object (and trackable by the tracking system) to track an object pose of image data of the object in a virtual space. The method may comprise tracking the second tracker to track a position of the centre point in the virtual space based on the position of the centre point relative to the second tracker. Further still, the method may comprise generating first display instructions for displaying in the virtual space, based on the object pose of the image data of the object in the virtual space, the position of the centre point in the virtual space and the radius, a visual representation indicative of the radius relative to the image data of the object.

As understood herein, the display instructions are configured to control a display such that certain information is visually output to a user. As such, the display instructions will typically take the form of analog or digital signals.

The visual representation indicative of the radius may have been generated (e.g., scaled) such that the display properly reflects the actual location of the spherically shaped surface portion relative to the actual location of the object (e.g., relative to a surface portion of the object). As such, the image data of the object and the radius indication may be converted to a common scale for visualization.

The calibration structure may be configured to centre the centre point onto a central axis of the calibration structure. The object may be a surgical object, such as a second instrument or a patient. The image data of the object may be captured using a conventional camera (e.g., a stereo camera), computer tomography, magnetic resonance imaging or any other medical imaging procedure.

The tracking system may be configured to apply one or more of an optical tracking technique, an electromagnetic tracking technique and an ultrasound tracking technique. The optical tracking technique may rely on one or both of infrared and visible light. The initial calibration steps may be performed using an optical tracking technique and the later tracking steps may be performed using an electromagnetic tracking technique, or vice versa.

The method may comprise determining the radius by multiplying the sinus of half of the opening angle with a distance between the position of the centre point and a real or imaginary point relative to which the opening angle is defined.

The calibration device may comprise a plurality of calibration structures, wherein the first tracker is arranged in a predetermined manner relative to each of the plurality of calibration structures. The method may further comprise determining the position and, optionally, the opening angle of the calibration structure in which the instrument tip is arranged by identifying the calibration structure based on the position of the centre point. The central axis of at least two calibration structures may be arranged parallel to each other. The central axis of at least two calibration structures may alternatively be arranged non-parallel to each other, such as at an angle of 90°.

The positional data may comprise at least two different tilt poses of the second tracker for the different tilting positions. Alternatively, or additionally, the positional data may comprise at least four different positions of the second tracker for the different tilting positions.

The method may comprise receiving an expected radius value and validating whether the determined radius corresponds to the expected radius value. The validation may be performed based on a threshold difference between the expected radius value and the determined radius. The method may comprise receiving the expected radius value as user input (e.g., entered via a keyboard or graphical user interface). The method may further comprise generating display instructions for displaying a result of the validation.

The method may further comprise outputting, on a display, instructions to tilt the instrument for calibration purposes. The instructions may indicate when to start and/or to stop tilting the instrument.

The method may comprise generating region display instructions for displaying a non-target region in the image data for which contact with the instrument is to be avoided. The method may comprise outputting a warning when the instrument tip comes in contact with the non-target region and/or a threshold region around the non-target region. The warning may be at least one of an acoustic, optical, and haptic warning.

The method may comprise generating progress display instructions for displaying a progress of object manipulation based on a path of the instrument tip. The progress of object manipulation may be based on a surface generated from a circle or sphere with the determined radius and a tracked movement of the centre point as a generatrix.

The method may comprise displaying, on a display, a visual representation (e.g., an icon) of at least a portion of the instrument relative to the image data of the object. The display may be part of the tracking system, a surgical navigation system or be a separate display that is communicatively coupled with the tracking system or the surgical navigation system.

The calibration structure may have a shape of a cone or a pyramid, or a portion thereof, defining the opening angle. The calibration structure may comprise plates or other rigid structures that form a (e.g., partial) cone or partial pyramid. The calibration structure may have a shape of a truncated cone or pyramid.

The method may further comprise determining from a pre-determined list of radii a subset of (one or more) radii that meet a similarity criterion with respect to the determined radius. The similarity criterion may comprise having a radius that differs by not more than a difference threshold from the determined radius. Additionally, or alternatively, the similarity criterion may comprise a number of radii that are closest to the determined radius. The method may further comprise generating an output for a user selection or confirmation, wherein the output comprises at least one of information about the subset of radii and information about instrument tips associated with the subset of radii. The user may thus select, or confirm, a particular instrument tip attached to or inserted into the (e.g., powered) instrument.

The visual representation may comprise an icon representative of the centre point and at least a part of a circle or a sphere with the determined radius of the spherically shaped surface portion around the icon. The icon may further be representative of the centre point with the determined radius of the spherically shaped surface portion around the centre point. The icon may be representative of an actual instrument tip (e.g., as selected or confirmed by a user).

A pose of an instrument axis may be determined on the basis of tracking the second tracker while the instrument is rotated about the instrument axis. Alternatively, the pose of the instrument axis may be determined on the basis tracking the second tracker and evaluating a known predetermined spatial relationship between the second tracker and the instrument axis. The visual representation may be indicative of the pose of the instrument axis in the virtual space. The instrument axis may be represented in the virtual space as a line that extends towards the centre point of the spherically shaped surface portion.

According to a third aspect, a computer program product is provided, comprising instructions that, when executed on at least one processor, cause the at least one processor to carry out any of the methods described herein. The computer program product may be stored on a non-volatile memory, such as a hard disc drive, a flash memory, a read-only memory, or an optical disc.

According to a fourth aspect, a device for determining a radius of a spherically shaped surface portion of a tip of an instrument is provided. A calibration device is provided that comprises a flat calibration surface and a calibration structure, wherein the calibration structure defines an opening angle and a real or imaginary first reference point relative to which the opening angle is defined. The calibration device comprises a first tracker trackable by a tracking system and arranged in a first predetermined relationship relative to the first reference point and in a second predetermined relationship relative to the calibration surface, and wherein the instrument comprises a second tracker trackable by the tracking system. The device is configured to determine a first pose of the first tracker and a second pose of the second tracker while the spherically shaped surface portion is in abutment with the calibration structure. The device is further configured to determine a reference position of the first reference point relative to the second tracker when the first tracker is in the first pose and the second tracker is in the second pose based on the first pose, the second pose, and the first predetermined relationship. The device is configured to determine a third pose of the first tracker and a fourth pose of the second tracker while the spherically shaped surface portion is in abutment with the calibration surface. The device is further configured to determine the radius of the spherically shaped surface portion based on the third pose, the second predetermined relationship, the reference position, the fourth pose, and the opening angle.

According to a fifth aspect, a device for determining a radius of a spherically shaped surface portion of a tip of an instrument is provided. The spherically shaped surface portion has a centre point. A calibration device is provided that comprises a calibration structure defining an opening angle and that is configured to cooperate with the spherically shaped surface portion so as to guide a tilting movement of the instrument tip around the centre point. The calibration device comprises a first tracker trackable by a tracking system and arranged in a predetermined relationship relative to the calibration structure and wherein the instrument comprises a second tracker trackable by the tracking system. The device is configured to determine a first pose of the first tracker. The device is further configured to determine positional data of the second tracker for different tilting positions of the instrument while the spherically shaped surface portion is in abutment with the calibration structure. The device is configured to determine a position of the centre point relative to the second tracker based on the positional data. The device is configured to determine the radius of the spherically shaped surface portion based on the position of the centre point, the first pose, the predetermined relationship, and the opening angle.

The calibration structure may define a real or imaginary first reference point relative to which the opening angle is defined and the predetermined relationship may define a position of the first reference point relative to the first tracker.

The device of the fourth or fifth aspect may be configured to perform any of the method steps described herein. The device may be a computer system or a part of a computer system.

According to one example, a calibration system is provided. The calibration system comprises the device for determining a radius as described herein. The calibration system may further comprise the calibration device with the first tracker coupled thereto and the instrument with the second tracker coupled thereto.

According to another example a surgical navigation system is provided. The surgical navigation system comprises the calibration system as described herein. The surgical navigation system further comprises the tracking system, wherein the tracking system comprises or is communicatively coupled to the device for enabling a radius determination.

According to another example, a method for enabling a visualization of an indication of a radius of a spherically shaped surface portion of a tip of an instrument relative to image data of an object in a virtual space is provided. The spherically shaped surface portion has a centre point. A calibration device is provided that comprises a calibration structure defining an opening angle and that is configured to cooperate with the spherically shaped surface portion so as to guide a tilting movement of the instrument tip around the centre point. The calibration device comprises a first tracker trackable by a tracking system and arranged in a predetermined relationship relative to the calibration structure. The instrument comprises a second tracker and the object comprises a third tracker trackable by the tracking system. The method is performed by a computer system and comprises determining a first pose of the first tracker. The method further comprises determining positional data of the second tracker for different tilting positions of the instrument while the spherically shaped surface portion is in abutment with the calibration structure. The method also comprises determining the radius of the spherically shaped surface portion and a position of the centre point relative to the second tracker based on the first pose, the positional data, the predetermined relationship, and the opening angle. The method comprises tracking the third tracker to track a second pose of the image data of the object in the virtual space. The method comprises tracking the second tracker to track a position of the centre point in the virtual space based on the position of the centre point relative to the second tracker. Further still, the method comprises generating first display instructions for displaying in the virtual space, based on the second pose of the image data of the object in the virtual space, the position of the centre point in the virtual space and the radius, a visual representation indicative of the radius relative to the image data of the object.

**Brief Description of the Drawings**

Further details, advantages and aspects of the present disclosure will become apparent from the following embodiments when taken in conjunction with the drawings, wherein:

Fig. 1A      shows an instrument tip with an end point as virtual representation of an instrument tip;

Fig. 1B      shows the instrument tip of Fig. 1A cutting an opening into tissue;

Fig. 1C      shows the instrument tip of Fig. 1A with an instrument axis oriented in a non-perpendicular angle relative to the surface of tissue;

Fig. 1D      shows the instrument tip in the orientation of Fig. 1C cutting an opening into the tissue;

Fig. 2A      shows a visual guidance comprising image data of tissue and a visual representation of an instrument in form of an end point;

Fig. 2B      shows the actual tissue with the corresponding actual location of the instrument tip in the scenario of Fig. 2A;

Fig. 3      shows a surgical navigation system comprising a device embodiment;

Fig. 4A-C      show enlarged views of spherically shaped instrument tips;

Fig. 5A      shows a calibration device with a first tracker and a calibration structure defining an opening angle;

Fig. 5B      shows a calibration device for electromagnetic tracking, wherein the first tracker comprises coils;

Fig. 6A      shows the instrument tip arranged inside a calibration structure with the shape of a cone;

Fig. 6B      shows a top view of the cone of the calibration structure shown in Fig. 6A and a circular contact region;

Fig. 6C      shows a calibration structure with an inverted regular pyramid with three sides;

Fig. 6D      shows a top view of the pyramid of the calibration structure shown in Fig. 6C with three contact regions;

Fig. 7A      shows a calibration structure with an inverted regular pyramid with six sides;

Fig. 7B      shows a top view of the pyramid of the calibration structure shown in Fig. 7A with six contact regions;

Fig. 8      shows a flow diagram of a method for visualizing an indication of a radius of a spherically shaped surface portion of an instrument tip;

Fig. 9A      shows three different tilting positions of the surgical instrument while the spherically shaped surface portion is in abutment with the calibration structure;

Fig. 9B      shows a side view of the spherically shaped surface portion in abutment with the calibration structure;

Fig. 10      shows geometrical relationships between the tracking system, the surgical instrument, and the calibration structure;

Fig. 11      shows an enlarged view of the spherically shaped surface portion in abutment with the calibration structure and geometric parameters relevant for determining the radius;

Fig. 12A      shows a further calibration device;

Fig. 12B      shows a still further example of the calibration device;

Fig. 13      shows a flow diagram of an alternative method for determining a radius of the spherically shaped surface portion of an instrument tip;

Fig. 14A      shows the spherically shaped surface portion in abutment with the calibration structure;

Fig. 14b      shows the geometrical relationship between components depicted in Fig. 14A;

Fig. 15A      shows the spherically shaped surface portion in abutment with the calibration surface;

Fig. 15B      shows the geometrical relationship between components depicted in Fig. 15A;

Fig. 16A      shows geometric relationships between the radius, a first reference point, the spherically shaped surface portion, and the opening angle;

Fig. 16B      shows a reference distance related to the calibration surface and a second reference point;

Fig. 17      shows a geometrical relationship between the second reference point, the calibration surface, the radius, and the centre point;

Fig. 18A      shows an example of the calibration device, wherein a central axis of the calibration structure is arranged parallel to a surface normal of the calibration surface;

Fig. 18B      shows an example of the calibration device, wherein the central axis is not arranged parallel to the surface normal;

Fig. 18C      shows the calibration device of Fig. 18A, wherein an orientation of an instrument tip is identical for two different poses of an instrument tracker;

Fig. 18D      shows the calibration device of Fig. 18B, wherein the orientation of the instrument tip does not change relative to a central axis and a surface normal;

Fig. 18E          shows the calibration device of Fig. 18A, wherein an orientation of the instrument tip changes relative to the central axis and the surface normal;

Fig. 18F          shows the calibration device of Fig. 18B, wherein a reorientation angle and a surface angle are different from zero;

Fig. 19           shows a geometrical relationship between the radius, the reference distance and a pivot angle;

Fig. 20A          shows a geometrical relationship of the second reference point, the centre point, a reference axis, and the pivot angle;

Fig. 20B          shows a reconstruction of the radius around the centre point;

Fig. 21           shows an enlarged view of two trackers being tracked by a tracking system;

Figs. 22A-C       show virtual space examples each with a visual representation indicative of a radius being arranged relative to image data of the object;

Fig. 23A          illustrates a subset of radii that meet a similarity criterion with a difference threshold;

Fig. 23B          shows a display output displaying a subset of radii highlighted within a pre-determined list of radii;

Fig. 23C          shows a display output displaying a subset of radii with information about the subset of radii and information about instrument tips associated with the subset of radii;

Fig. 23D          shows a display output displaying a subset of radii with information about the subset of radii, wherein some instrument tips have the same radius, but differ in a tip shape;

Fig. 24A          shows a fourth example of a virtual space with an instrument model as a visual representation indicative of the radius;

Fig. 24B          shows a fifth example of a virtual space with an instrument model icon as a visual representation;

Fig. 24C          shows a sixth example of a virtual space, wherein the visual representation comprises a three dimensional model of the surgical instrument; and

Fig. 25           shows a calibration device with a first calibration structure and a second calibration structure.

## Detailed Description

In the following description of exemplary embodiments, the same reference numerals are used to denote the same or similar components.

The embodiments described hereinafter are directed to determining a radius of a spherical surface of an instrument tip. The instrument may be used in various technical fields such as in robotics, material science, or medical procedures. The following embodiments exemplarily relate to surgical navigation of a surgical instrument.

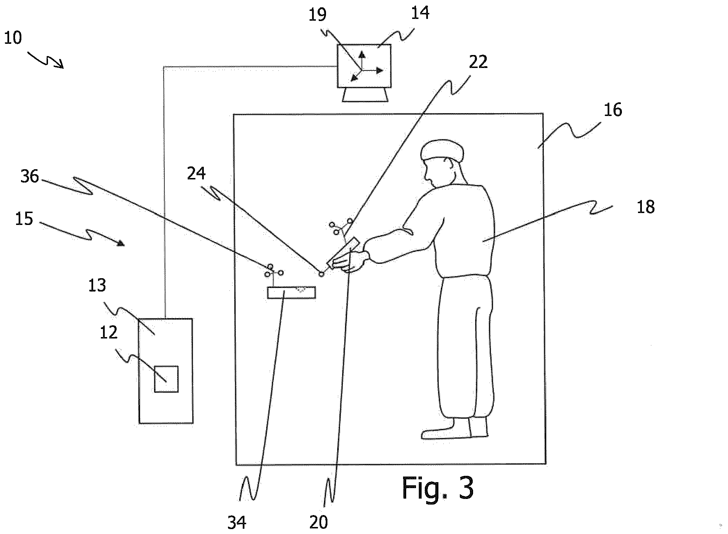

Fig. 3 shows an embodiment of a surgical navigation system 10 comprising a device 12 configured for determining the radius of a spherical surface of an instrument tip. The device 12 shown in Fig. 3 may be comprised by a dedicated computer system 13. Alternatively, or in addition, the device 12 may by comprised by cloud computing resources.

The surgical navigation system 10 further comprises a tracking system 14. The tracking system 14 shown in Fig. 3 comprises a camera (e.g., a stereo camera) for optical tracking. Additionally, or alternatively, the tracking system 14 comprises an electromagnetic field generator for electromagnetic tracking. The tracking system 14 is configured to gather tracking data within a surgical environment 16. The tracking data may take the form of images of the camera or electrical currents induced in an electromagnetic tracker (e.g., in one or more coils).

Fig. 3 also illustrates a user 18 (e.g., a surgeon or medical staff) moving a surgical instrument 20 relative to a calibration device 34 in the surgical environment 16. The associated calibration procedure targets at determining a tip radius of the surgical instrument 20. The device 12, the calibration device 34 and the surgical instrument 20 are part of a calibration system 15 comprised by the surgical navigation system 10.

As shown in Fig. 3, the calibration device 34 comprises a first tracker 36 and the surgical instrument 20 comprises a second tracker 22. The first and second trackers 36, 22 are both trackable by the tracking system 14. The first and second trackers 36, 22 exemplarily shown in Fig. 3 are passive trackers with reflective markers (e.g., reflective spheres). Alternatively, the first and second trackers 36, 22 may be active trackers with light emitting elements. Further alternatively, the first and second trackers 36, 22 may comprise coils for electromagnetic tracking. As a still further alternative, the surgical instrument 20 and the calibration device 34 as such may also constitute respective trackers 36, 22. For example, using a stereo camera of the tracking system 14, the tracking system 14 may simply track the known shapes of the one or both of the surgical instrument 20 and calibration device 34 for determining their positions or poses within the surgical environment 16.

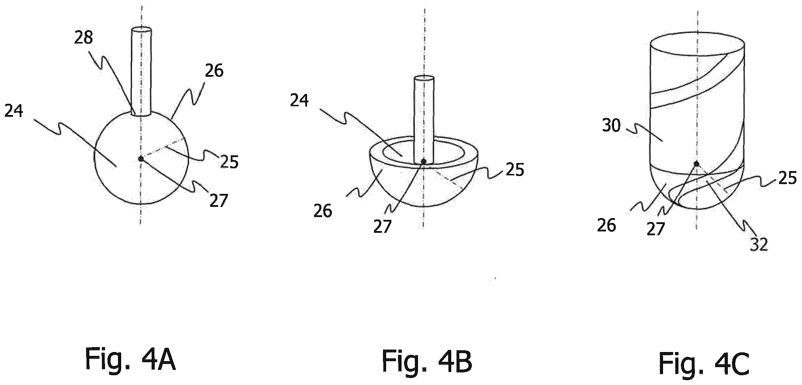

Fig. 4A shows an enlarged view of an exemplary instrument tip 24 of the surgical instrument 20. The instrument tip 24 has a radius 25 that is defined by a spherically shaped surface portion 26 around a centre point 27. The surface portion 26 may have openings or rake structures (e.g., in the form of cutting flutes) and may therefore not form one continuous surface. In some variants, the instrument tip 24 may be a burr.

The surface portion 26 of the instrument tip 24 shown in Fig. 4A forms almost a complete sphere with the exception of an attachment region 28 where the tool tip 24 is connected to a shaft of the surgical instrument 20. The shaft with the tool tip 24 may be removable from the surgical instrument 20, so that the user 18 may selectively use the surgical instrument 20 with different tool tips 24 having different radii.

Alternatively to the example illustrated in Fig. 4A, the spherically shaped surface portion 26 may form a smaller portion of a sphere. Fig. 4B shows an instrument tip 24 with a surface portion 26 that forms a half sphere. The surface portion 26 may form any other portion of a sphere such as a third, or a quarter of a sphere. Fig. 4C shows an instrument tip 24 in the form of a drill bit with an essentially spherically shaped surface portion 26 and an essentially cylindrical surface portion 30. The instrument tip 24 comprises one or more grooves 32 that interrupt a continuous surface of the spherically shaped surface portion 26.

In the examples illustrated in Figs. 4A to 4C, the spherically shaped surface portion 26 defines a virtual sphere with the radius 25 and the centre point 27. Points on the surface portion 26 are arranged with a distance of the radius 25 away from the common centre point 27. Prior to actual surgery, the radius 25 of the spherically shaped surface portion 26 will need to be determined by the user 18 using the calibration device 34.

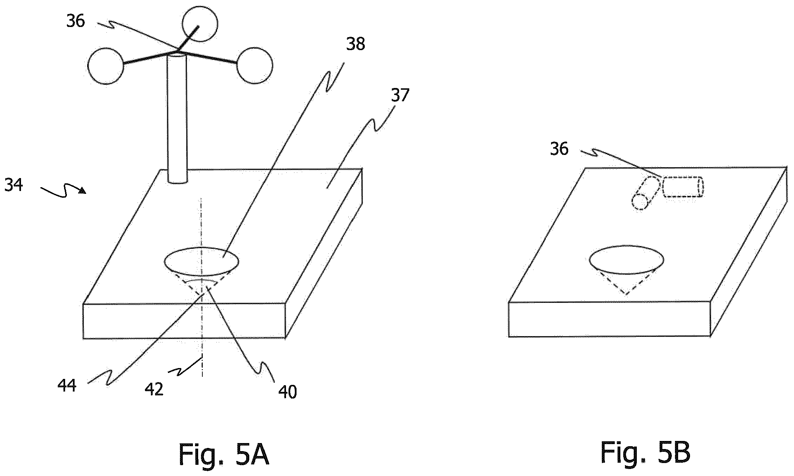

Fig. 5A shows an example of the calibration device 34 with the first tracker 36 and a calibration structure 38 defining an opening angle 40. The first tracker 36 needs to be trackable by the tracking system 14 of the surgical navigation system 10. Therefore, in the above example of a camera-based optical navigation system 10, the first tracker 36 is an optical tracker as shown in Fig. 5A with three (or more) reflective spheres. In case the surgical navigation system 10 employs electromagnetic tracking, the first tracker 36 may comprise one, two or more coils as shown in Fig. 5B.

The calibration structure 38 shown in Fig. 5A is formed as a cone-shaped surface opening in a plate-shaped body 37. The resulting cone 38 defines a central axis 42 through an apex 44 of the cone 38. The cone 38 has an opening angle 40 that is defined as twice the angle between the central axis 42 and any surface line of the cone 38 lying in the same plane as the central axis 42. The opening angle 40 may also be defined as the largest angle between two surface lines of the cone 38 lying in the same plane as the central axis 42. The opening angle 40 may be between 30° and 150°, such as between 80° and 100°. The opening angle may for example, be selected to be 45°, 90° or 120°.

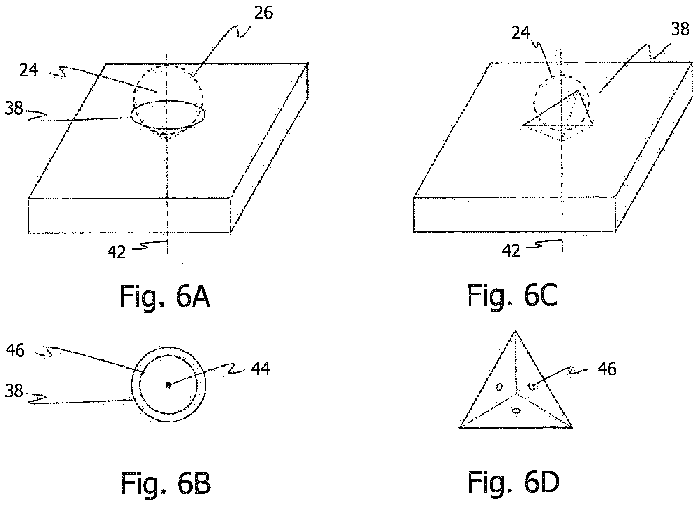

Fig. 6A shows the instrument tip 24 arranged inside the cone-shaped calibration structure 38 of Fig. 5A. As shown in Fig. 6B, the cone 38 is a circular cone, which means a plane perpendicular to the central axis 42 intersects the cone not in an oval but a circle. Consequently, the spherically shaped surface portion 26 abuts against the cone 38 in a circular contact region. Fig. 6B shows a top view of the cone 38 shown in Fig. 6A and the circular contact region 46. In the top view, a centre of the circular contact region 46 aligns with the apex 44 and therefore with the central axis 42. Due to the rotational symmetry of the contact region 46 and the spherically shaped surface portion 26, the centre point 27 of the spherically shaped surface portion 26 is centred by the cone 38 onto the central axis 42. The centre point 27 is arranged on the central axis 42 independently of the radius 25 of the surface portion 26 (as long as the surface portion 26 fits into the cone 38). However, the radius 25 correlates with a distance of the centre point 27 relative to the apex 44 of the cone 38. A smaller radius 25 of the surface portion 26 results in a shorter distance between the centre point 27 and the apex 44 compared to a larger radius 25 of the surface portion 26 that results in a larger distance between the centre point 27 and the apex 44.

The centring function of the calibration structure 38 does not necessarily require a cone shaped opening. Alternatively, the calibration structure 38 can have an opening with the shape of a regular pyramid, which means that a plane perpendicular to a central axis of the pyramid intersects the pyramid in a regular polygon (with equal side lengths and inner angles). As an example, the pyramid can have three, four, five, or more sides. Fig. 6C shows a pyramidal calibration structure 38 with an inverted regular pyramid with three sides. Fig. 6D shows a top view of the pyramid of the calibration structure 38 and three contact regions 46.

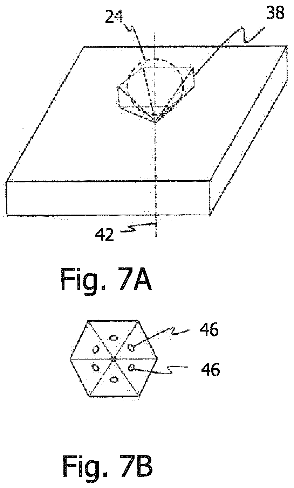

Fig. 7A shows another example of a pyramidal calibration structure 38 with an inverted regular pyramid with six sides. Fig. 7B shows a top view of the pyramid of the calibration structure 38 and six contact regions 46. The contact regions of the pyramid are positioned along a circle with a centre point that is arranged on a central axis 42 of the pyramid. For a pyramid, the opening angle is defined as twice the angle between a pyramid side and the central axis 42. In case of a pyramid with an even amount of sides, the opening angle is also the angle between two opposite pyramid sides.

A calibration structure 38 in the shape of a cone or a pyramid with at least three sides, such as described above, results in a circular contact region or at least three contact regions that confine the centre point 27 of the spherically shaped surface portion 26 onto a single point.

The various calibration structures 38 described herein are capable of receiving the instrument tip 24 and are configured to cooperate with the spherically shaped surface portion 26 so as to guide a tilting movement of the instrument tip 24 around the centre point 27 of the spherically shaped surface portion 26. In the process of guiding the tilting movement, the spherically shaped surface portion 26 slides along the calibration structure 38. Therefore, the calibration structure 38 preferably comprises a hard material with a Young's modulus larger than 150 GPa such as ceramics or metal (e.g., stainless steel).

As can be seen if Figs. 3 and 5A, the tracker of the calibration device 34 is arranged in a predetermined relationship relative to the calibration structure 38. The predetermined relationship may be defined by a pose (i.e., orientation and position) or position of the apex 44 relative to the first tracker 36 of the calibration structure. The predetermined relationship may, for example, be defined as a matrix transformation or vector in a coordinate system 19 (see Fig. 3) of the tracking system 14 that maps a pose or position of the apex 44 onto the first tracker 36 (or vice versa). Alternatively, or additionally, the surgical navigation system 10 may have access to geometrical properties (e.g., a digital model) of the calibration device 34.

As will be described below, the calibration device 34 and similar calibration devices can be used for determining the radius 25 of the spherically shaped surface portion 26. Based on the radius 25 thus determined, in optional subsequent steps, display instructions can then be generated during a later surgical procedure to cause a display to output a visual representation indicative of the determined radius 25 relative to image data of a patient.

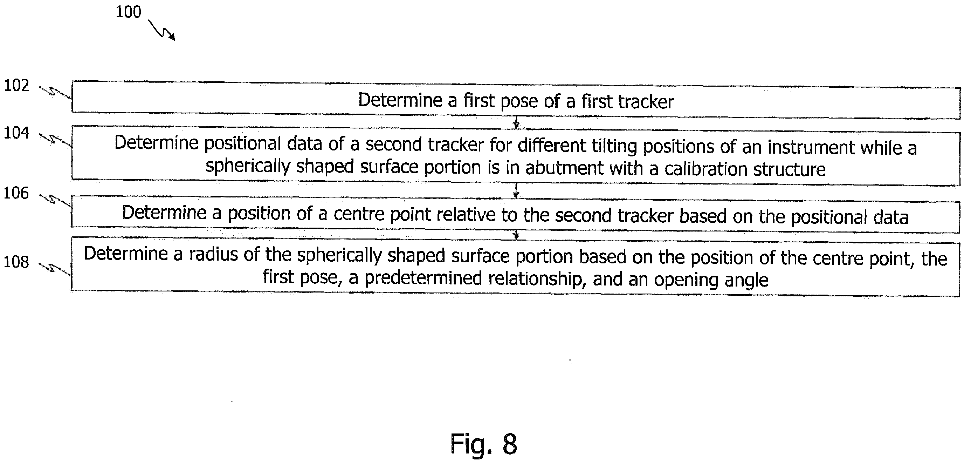

Fig. 8 shows a flow diagram 100 of a first embodiment of a computer-implemented method for determining a radius 25 of the spherically shaped surface portion 26 of the tip 24 of the surgical instrument 20 using the calibration device 34.

The method comprises determining, in step 102, a pose of the first tracker 36 as associated with the calibration device 34. The pose of the first tracker 36 comprises a position and orientation of the first tracker 36 and may be determined in the coordinate system 19 of the tracking system 14. Any technique known in the prior art may be used for determining the pose of the tracker 36. For optical tracking, a common approach is to capture one or more images of the reflective markers of the tracker 36 and determine the pose based on a known geometric arrangement of the markers relative to each other (Fig. 5A). For electromagnetic tracking, a common approach is for a field generator of the surgical navigation system 10 to generate an electric field that induces currents in coils constituting the tracker 36 (Fig. 5B). Based on a measurement of the induced currents, the pose of the first tracker 36 may be determined.

The method further comprises determining, in step 104, positional data of the second tracker 22 associated with the surgical instrument 20, referred to as instrument tracker 22 hereinafter (see Fig. 3), for different tilting positions of the surgical instrument 20 while the spherically shaped surface portion 26 remains in abutment with the calibration structure 38. The positional data may comprise at least two different tilting poses of the instrument tracker 22 for the different tilting positions and/or at least four different positions of the instrument tracker 22 for the different tilting positions.

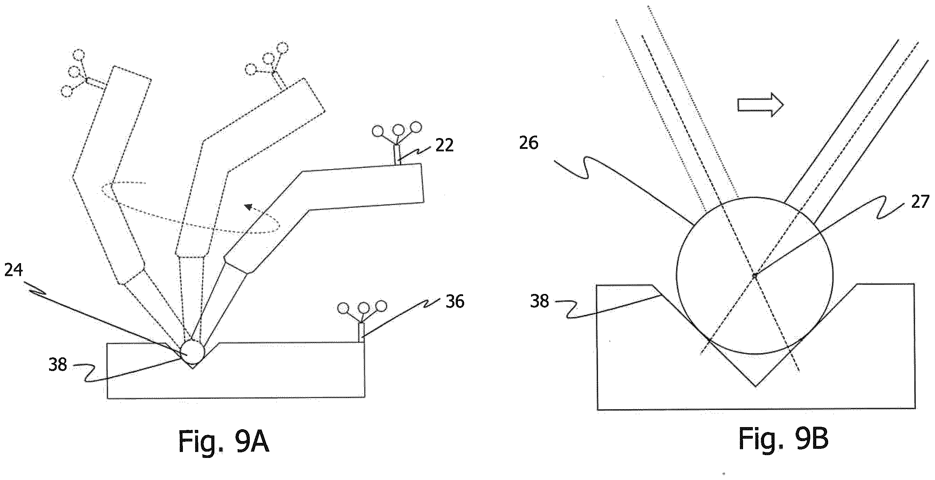

Fig. 9A shows examples of three different tilting positions of the surgical instrument 20 while the spherically shaped surface portion 26 remains in abutment with the calibration structure 38. The different tilting positions of the surgical instrument 20 cause the instrument tracker 22 to move along a sphere around the centre point 27. The position and orientation of the first tracker 36 coupled to the calibration device 34 is, on the other hand, not affected by the different tilting positions of the surgical instrument 20.

The method optionally comprises generating user instructions for displaying on a display of the surgical navigation system 10 (or of another computing device communicatively connected with the surgical navigation system 10) instructions to tilt the surgical instrument 20 for calibration purposes. Such user instructions will initially request the user 18 to start moving the surgical instrument 20 for acquisition of pose or position data and will inform him or her once sufficient data for radius calculation have been acquired.

The method further comprises determining, in step 106, a position of the centre point 27 relative to the second tracker 22 based on the positional data.

According to geometrical principles, the radius 25 can be determined based on the position of the centre point 27 of the instrument tip 24. The positional data acquired for the instrument tracker 22 can be used in this regard to determine a position of the centre point 27 of the spherically shaped surface portion 26. Depending on whether the positional data comprises poses of the instrument tracker 22 or only positions of the instrument tracker 22, two different approaches can be performed in order to determine the position of the centre point 27.

In the first approach for determining the position of the centre point 27, the positional data comprises at least two different poses of the instrument tracker 22 for the different tilting positions. Fig. 9B shows a side view of the spherically shaped surface portion 26 in abutment with the calibration structure 38. While the instrument tip 24 remains in abutment with calibration structure 38, the calibration structure 38 guides tilting of the instrument tip 24 (and, thus, of the surgical instrument 20 and the instrument tracker 22) around the centre point 27. Therefore, two different poses of the instrument tracker 22 for two different tilting positions differ (at least partly) in a rotation around the centre point 27. The position of the centre point 27 may be determined based on a transformation comprising a rotational transformation that maps one of the two poses onto the other pose. The position of centre point 27 is a position of unity (i.e., the only position unaltered by the transformation) of the rotation transformation.

Another way to determine the position of the centre point 27 based on the two poses is to determine for each marker of the instrument tracker 22 a linear vector that translates the marker between the two poses. For each translation vector a halfway point is further determined that is arranged in the centre between the positions of the marker for each of the poses. Due to the poses being arranged on a sphere, a connection vector from the halfway point to the (yet unknown) centre point 27 is arranged orthogonally to the direction of the translation vector. Therefore, a scalar product between the connection vector and the translation vector is zero. This allows defining, for each reflective marker of the instrument tracker 22, an equation that includes the position of the centre point 27 (in form of the connection vector). These equations can be solved for the position of the centre point 27.

It should be noted that the calculations described above require at least two poses of the instrument tracker 22. To increase the overall precisions, more than two poses may be captured, and determining the position of the centre point 27 may be performed with the more than two poses. A final position of the centre point 27 may be calculated from an average of multiple positions of the centre point 27 determined for the more than two poses of the instrument tracker 22. Determining the centre point 27 on the basis of more than two poses generally decreases a statistical error of the calculation.

In the second approach, the positional data comprises at least four different positions of the instrument tracker 22 for the different tilting positions. When the surgical instrument 20 is tilted around the centre point 27, the position of a centre of the instrument tracker 22 is moved on a sphere around the centre point 27. A sphere can be defined by, and reconstructed from, four points of the sphere (not arranged in a common plane). Therefore, the four positions of the instrument tracker 22 contain enough information for determining the sphere and, consequently, the position of its centre point 27. One way to determine the centre point 27 is to set up equations that define a distance (e.g., via Pythagorean equations) between the four tracker positions relative to the unknown point centre 27 and equate the distance to an unknown radius. The four equations include four unknown variables (i.e., three coordinates of the position of the centre point 27 and the radius) and therefore form a determined system that is mathematically solvable. It should be noted that the radius of the sphere in these equations is not the radius of the spherically shaped surface portion 26, but the distance between the centre point 27 and the centre of the instrument tracker 22. In order to reduce the statistical error of the calculation, the position of the centre point 27 may of course be determined based on more than four positions of the instrument tracker 22.

It should be noted that both approaches are not exclusive to each other. For example, in the case that the positional data comprise more than three poses of the instrument tracker 22, both approaches can be applied simultaneously. The position of centre point 27 may be determined from the positions obtained by both approaches (e.g., applying an averaging algorithm).

The method further comprises determining, in step 108, the radius 25 of the spherically shaped surface portion 26 based on the position of the centre point 27, the first pose of the first tracker 36, the predetermined relationship between the first tracker 36 and the calibration structure 38, and the opening angle 40 of the calibration structure 38.

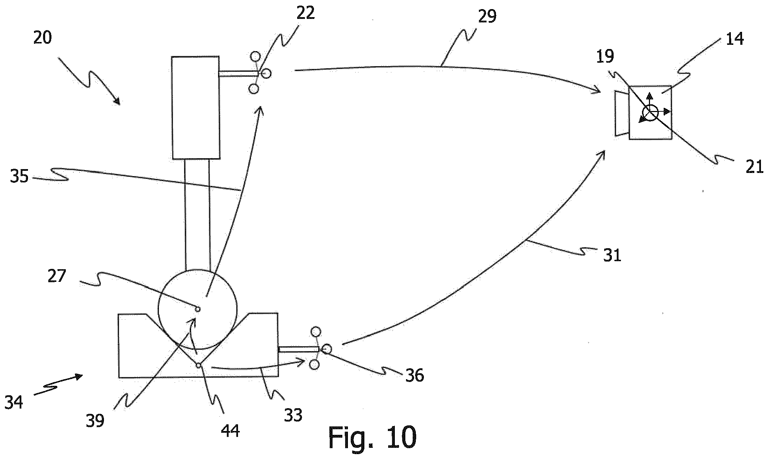

Based on the first pose of the first tracker 36 of the calibration device 34 and the predetermined relationship between the first tracker 36 and the calibration structure 38, a position of the apex 44 of the calibration structure 38 may be determined. Fig. 10 shows geometrical relationships between the tracking system 14, the surgical instrument 20, and the calibration structure 38. The geometrical relationships are in the following described as transformations that map one point in space onto another point in space. The transformations may, for example, be defined as mathematical transformations such as a matrix (for translating a pose or position) or a vector (for translating a position). It should be noted that any transformation as described herein may alternatively be defined as its inverse transformation.

The tracking system 14 is configured to determine a pose of the instrument tracker 22 in form of a first transformation 29 and a pose of the first tracker 36 of the calibration device 34 in form of a second transformation 31. The first and second transformations 29, 31 may be defined as transformations from centres of each of the two trackers 36, 22 to an origin 21 of the coordinate system 19 of the tracking system 14. The origin 21 may, for example, be positioned in an optical centre of the camera of the tracking system 14. In the case of a stereo camera with two camera units, the origin 21 may be positioned in the optical centre of one of the two camera units or positioned between the optical centres of the two camera units.

The relationship between the first tracker 36 of the calibration device 34 and the calibration structure 38 shown in Fig. 10 may be a geometric relationship as defined by a third transformation 33 that maps a position of the apex 44 onto the centre of that tracker 36. A fourth transformation 35 can be defined based on the determined position of the centre point 27 and a pose determined for the instrument tracker 22.

Further, a fifth transformation 39 can be defined that maps the position of the centre point 27 onto the apex 44. As can be seen in Fig. 10, the first, second, third, fourth, and fifth transformations 29, 31, 33, 35, 39 jointly describe a closed circle of transformations. Consequently, the fifth transformation 39 can be determined by a sum of the first, second, third, and fourth transformations 29, 31, 33, 35. Based on the determined fifth transformation 39, a centre distance 56 between the position of the apex 44 and the position of the centre point 27 can be calculated. The centre distance 56 thus calculated can be used to determine the radius 25, as described below.

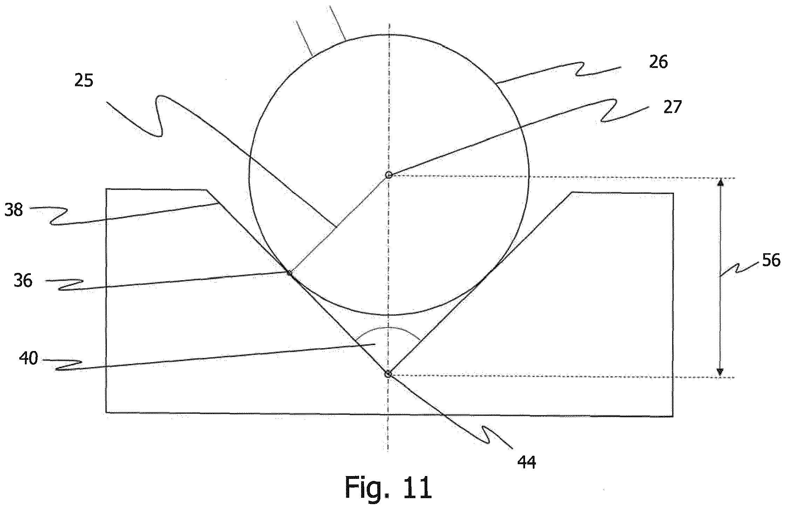

Fig. 11 shows an enlarged view of the spherically shaped surface portion 26 in abutment with the calibration structure 38 and geometric parameters relevant for determining the radius 25. As can be seen in Fig. 11, the radius 25 of the spherically shaped surface portion 26 can be calculated by multiplying the centre distance 56 with a sinus of half of the opening angle 40. It should be noted that in Fig. 11, the centre distance 56 can be defined as the distance between the centre point 27 and the apex 44, because the calibration structure 38 has an apex 44. In case the calibration structure 38 has a shape without an apex 44 such as a frustum (i.e., a truncated pyramid or cone), the centre distance 56 may generally be defined as a distance between the position of the centre point 27 and an imaginary point ("imaginary apex") relative to which the opening angle 40 is defined.

The first method embodiment illustrated in Fig. 8 in some implementations includes pivoting the surgical instrument 20 in the calibration structure 38 to arrive at different tilting positions, although the surgical instrument 20 could also be removed from the calibration structure 34 after a first tilting position has been recorded and placed therein a second time to record of a second tiling position, and so on. If pivoting is used to arrive the different tiling positions, there is a risk that a sharp instrument tip 24 will damage (e.g., cut into) the calibration structure 34. For this reason, in certain implementations a radius determination approach may be desired that does not necessarily rely on a pivoting instrument movement, as will now be described with reference to another calibration device example.

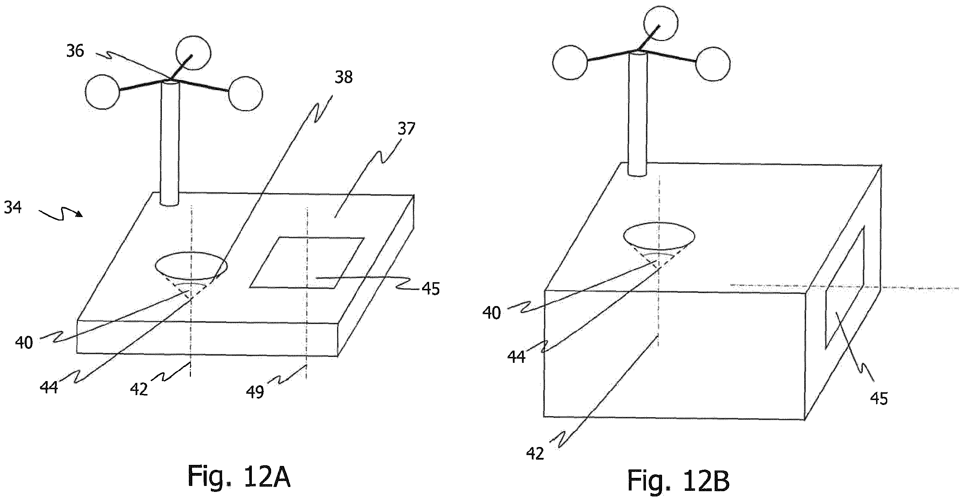

Fig. 12A shows a second example of a calibration device 34 with a first tracker 36 and a calibration structure 38 defining an opening angle 40 (as generally described above with reference to the first calibration device example). The calibration structure 38 shown in Fig. 12A is formed as a cone-shaped surface opening in a plate-shaped body 37. The calibration structure 38 defines a real or imaginary first reference point 44 relative to which the opening angle 40 is defined. In the example depicted in Fig. 12A, the calibration structure 38 is a cone and the first reference point 44 is located at the apex of the cone. In the case of a truncated cone, the first reference point 44 is located at a position where a non-truncated version of the truncated cone would have an apex. The calibration structure 38 has a central axis 42 that forms a bisector of the opening angle 40. The first tracker 36 is trackable by the tracking system 14 of the surgical navigation system 10.

The first tracker 36 is arranged in a first predetermined relationship relative to the first reference point 44. The first predetermined relationship may be defined by a position of the first reference point 44 relative to first tracker 36 of the calibration structure 38 (e.g., as described above with reference to Fig. 5A).

The calibration device 34 further comprises a flat calibration surface 45. The calibration surface 45 is configured for abutment with the spherically shaped surface portion 26 of the instrument tip 24. The calibration surface 45 defines a surface normal 49 that is arranged perpendicular to the calibration surface 45. For the calibration device 34 depicted in Fig. 12A, the central axis 42 and surface normal 49 are arranged parallel to each other. Alternatively, the central axis 42 and the surface normal 49 may be arranged at any other angle relative to each other, such as 30°, 45°, 90°, 120°, 180° (see the 90° example as depicted in Fig. 12B).

The calibration surface 45 is arranged in a second predetermined relationship relative to the first tracker 36 of the calibration device 34. The second predetermined relationship may comprise a transformation of the first tracker 36 onto a point of the calibration surface 45. For example, the second predetermined relationship may comprise (or define) a transformation of the pose of the first tracker 36 onto a centre point (or corner point) of the reference surface 45, wherein at the centre point is defined a vector perpendicular to the reference surface 45 or a coordinate system defining two (non-parallel) axes that are arranged parallel to the reference surface 45. An intermittent geometrical relationship between the calibration surface 45 and the first reference point 44 may be provided. The second predetermined relationship may then be determined based on the first predetermined relationship and the intermittent geometrical relationship.

The first example of the calibration device 34 depicted in Fig. 5A and the second example of the calibration device 34 depicted in Figs. 12A and 12B essentially differ by the calibration surface 45. It should be noted that the first method embodiment is compatible with a calibration device 34 that comprises a calibration surface 45, as the calibration surface 45 does not prohibit any of the steps 102 to 108 of the method according to the first embodiment. Similarly, the calibration device 34 shown in Fig. 5A also comprises flat surfaces, such as a surface around the calibration structure 38, which can be used as calibration surface for the purpose of the second method embodiment. Therefore, the different examples of calibration devices 34 described herein are not necessarily exclusive to one of the method embodiments described therewith, but may be compatible with both method embodiments.

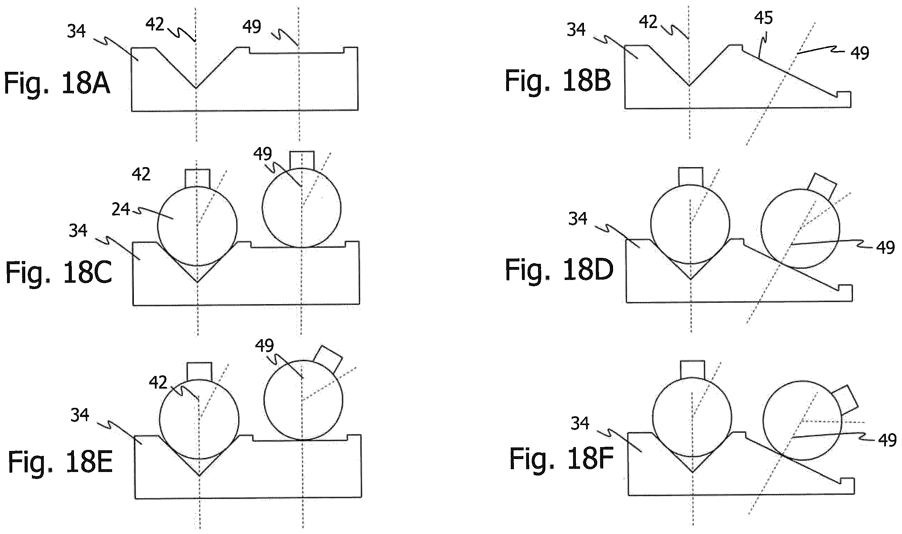

The calibration surface 45 may be a specific marking on the calibration device 34. The marking may be printed (e.g., as a circle, point, cross or, as illustrated in Figs. 12A and B, as a rectangle). The calibration surface 45 may be recessed into the plate-shaped body 37 of the calibration device (such as depicted in Fig. 18A) or may be at least partially surrounded by a wall in order to prevent an abutting tip 24 from slipping away from the calibration surface 45.

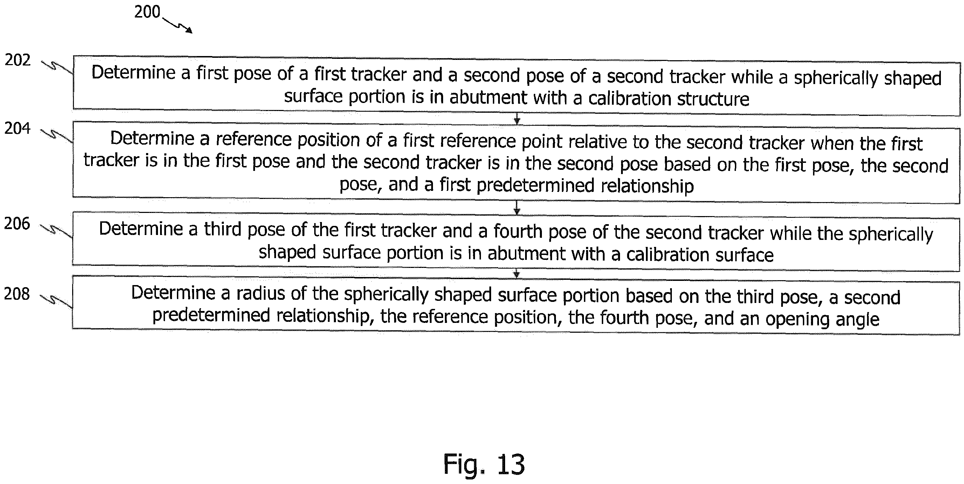

Fig. 13 shows a flow diagram 200 of a second embodiment of a computer-implemented method for determining a radius 25 of the spherically shaped surface portion 26 of the tip 24 of the surgical instrument 20 using the second example of the calibration device 34 as illustrated in Figs. 12A and B.

The method illustrated in Fig. 13 comprises determining, in step 202, a first pose of the first tracker 36 and a second pose of the second tracker 22 (subsequently called instrument tracker 22), while the spherically shaped surface portion 26 is in abutment with the calibration structure 38. Both poses may be determined substantially at the same point in time.

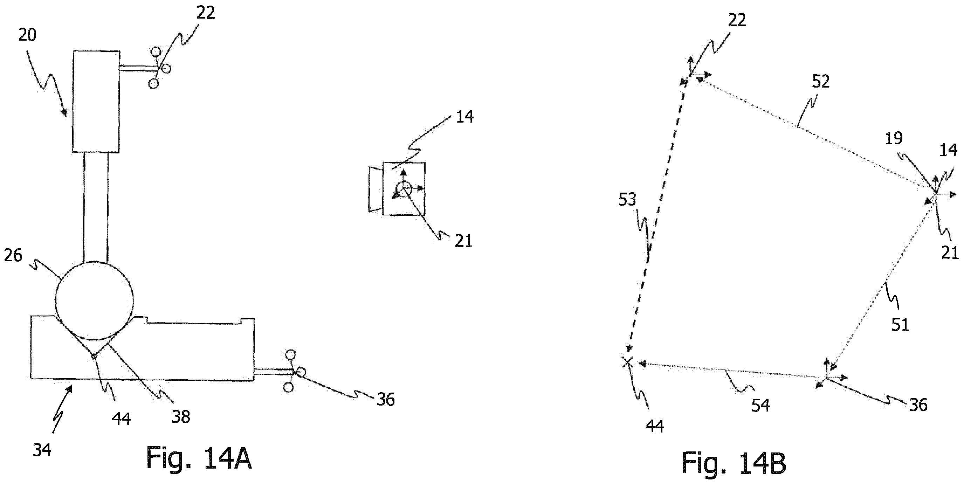

Fig. 14A shows the spherically shaped surface portion 26 in abutment with the calibration structure 38. Fig. 14B shows the geometrical relationship between the tracking system 14, the first tracker 36, the instrument tracker 22, and the first reference point 44. The tracking system 14 may be configured to determine the first pose of the first tracker 36 in form of a first transformation 51 and the second pose of the instrument tracker 22 in form of a second transformation 52. The first and second transformations 51, 52 may be defined as transformations from centres of each of the two trackers 36, 22 to an origin 21 of the coordinate system 19 of the tracking system 14. The origin 21 may, for example, be positioned in an optical centre of the camera of the tracking system 14. In the case of a stereo camera, the origin 21 may be positioned in the optical centre of one of the two camera units s or positioned between the optical centres of the two camera units.

The method further comprises determining, in step 204, a reference position 53 of the first reference point 44 relative to the instrument tracker 22 when the first tracker 36 is in the first pose and the second tracker 22 is in the second pose based on the first pose, the second pose, and the first predetermined relationship 54.

The position of the first reference point 44 can be located based on the first pose of the first tracker 36 and the first predetermined relationship. For example, the position of the first reference point 44 may be determined based on a combination of matrix transformations or vector sums of the first transformation 51 and the first predetermined relationship 54.

For example, in the case of translations in form of vectors and using the example depicted in Fig. 14B, the reference position 53 can be determined from a sum of vectors of the first transformation 51, the second transformation 52, and the first predetermined relationship 54. Considering the vector directions shown in Fig. 14B, the reference position 53 can be determined by subtracting a vector of the second transformation 52 from a sum of vectors of the first transformation 51 and the first predetermined relationship 54. Since a vector direction can generally be defined in either way, the algebraic signs in the sum changes with different definitions of vector directions.

The method further comprises determining, in step 206, a third pose of the first tracker 36 and a fourth pose of the instrument tracker 22 while the spherically shaped surface portion 26 is in abutment with the calibration surface 45. Both poses may be determined substantially at the same point in time.

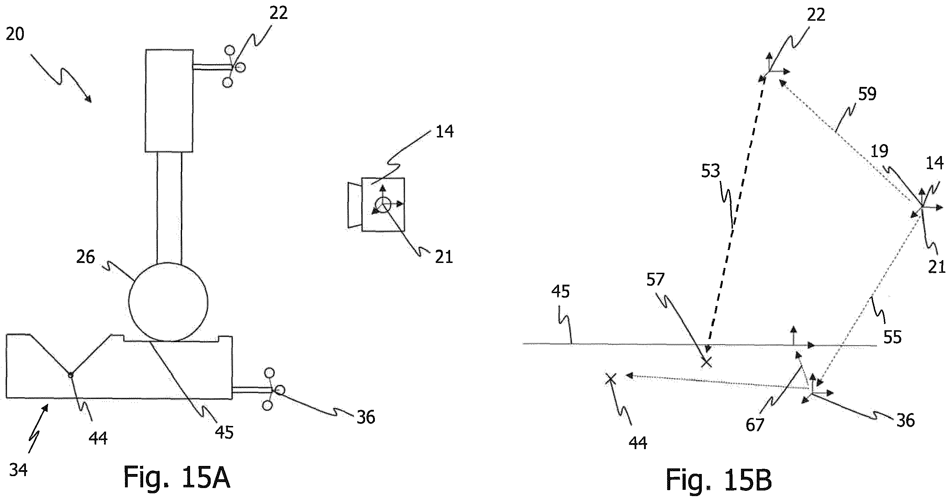

Fig. 15A shows the spherically shaped surface portion 26 in abutment with the calibration surface 45. Fig. 15B shows the geometrical relationship between the tracking system 14, the first tracker 36, the instrument tracker 22, the first reference point 44, a second reference point 57, the second predetermined relationship 67 and the reference surface 45. The tracking system 14 may be configured to determine the third pose of the first tracker 36 in form of a third transformation 55 and the fourth pose of the instrument tracker 22 in form of a fourth transformation 59.

It should be noted that the first pose and the third pose may be at least essentially identical. This may be the case, for example, when the calibration device 34 is fixedly arranged relative to the tracking system 14 (e.g., on a table) and the surgical instrument 20 is moved by the user relative to the calibration device 34. Similarly, the second and fourth pose of the instrument tracker 22 may be at least essentially identical. This may be the case, for example, when the surgical instrument 20 (for example in form of a robotic arm) is fixedly arranged relative to the tracking system 14 and the calibration device 34 is moved by the user relative to the calibration device 34. Furthermore, the first pose may not be identical to the third pose and the second pose may not be identical to the fourth pose. This may be the case, for example, when the surgical instrument 20 and the calibration device 34 are both manually moved relative to each other.

The method also comprises determining, in step 208, the radius 25 of the spherically shaped surface portion 26 based on the third pose, the second predetermined relationship 67, the reference position 53, the fourth pose, and the opening angle 40.

The third pose, the second predetermined relationship 67, the reference position 53, the fourth pose, and the opening angle 40 form five parameters that are sufficient for determining the radius 25. In the following, determining the radius 25 will be described in an intuitively accessible way. However, it should be understood, that the five parameters, or parameters derived therefrom, may be combined or processed in any other way that allows determining the radius 25.

The reference position 53 may be combined with the third pose in order to determine the second reference point 57. The second reference point 57 may be determined, for example, by a vector sum or matrix product of the fourth transformation 59 and the reference position 53. Alternatively, the first reference point 44 may be registered with the instrument tracker 22, while the instrument tracker 22 is in the second pose such that the registered first reference point 44 aligns with the second reference point 57 when the instrument tracker 22 is in the fourth pose.

The reference surface 45 can be constructed based on the third pose of the first tracker 36 and the second predetermined relationship 67. The reference surface 45 may be similarly reconstructed using a vector sum or matrix product of the third transformation 55 and the second predetermined relationship 67.

The radius 25 can be determined using a reference distance between the reference surface 45 and the second reference point 57. In order to better understand the correlation between the reference distance and the radius 25, the geometrical relationships of components involved in determining the radius 25 are discussed first.

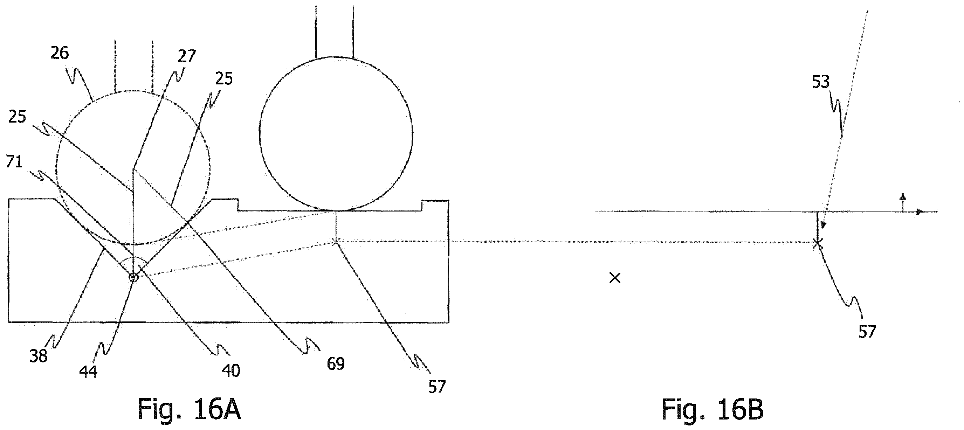

Fig. 16A shows geometric relationships between the radius 25, the first reference point 44, the spherically shaped surface portion 26, and the opening angle 40. The spherically shaped surface portion 26 abuts at an abutment point 69 against a surface of the calibration structure 38. The centre point 27, the abutment point 69, and the first reference point 44 form a triangle with a 90° angle at the abutment point 69 and half the opening angle 40 at the first reference point 44. The distance between the centre point 27 and the first reference point 44 is a sum of the radius 25 and a reference distance 71 between the first reference point 44 and (a closest point of) the spherically shaped surface portion 26. This geometrical relationship in the triangle can be described with the following equation:

$$ r = (r + d) \cdot \sin\left(\tfrac{\theta}{2}\right), \qquad\qquad \text{equation (1)} $$

with r as the radius 25, d as the reference distance 71, and $\theta$ as the opening angle 44. Solving equation (1) for r results in the following equation:

$$ r = \frac{d \cdot \sin\left(\tfrac{\theta}{2}\right)}{1 - \sin\left(\tfrac{\theta}{2}\right)} \qquad\qquad \text{equation (2)} $$

Since the opening angle 40 is one of the five known parameters, the radius 25 can be determined if the reference distance 71 is known. The reference distance 71 is defined by a distance between the first reference point 44 and the spherically shaped surface portion 26. Since the first reference point 44 and the second reference point 57 are both located at the reference distance 53 relative to the instrument tracker 22, the distance between the second reference point 57 and the spherically shaped surface portion 26 (when in abutment with the calibration surface 45) is the same as the reference distance 71. The equal distance is indicated by dashed lines in Fig. 16A.

As described above, the calibration surface 45 can be determined based on the third pose and the second predetermined relationship 67 and the second reference point 57 can be determined based on the fourth pose and the reference distance 53. Therefore, the reference distance 71 can be determined by a distance between the calibration surface 45 and the second reference point 57 as shown in Fig. 16B. The distance can be determined based on common mathematical equations for determining a distance between a plane and a point. One example is to define an arbitrary vector between the second reference point 57 and a point on the calibration surface 45, projecting the arbitrary vector onto a surface normal of the calibration surface 45 and determining the length of the projected vector. The radius 25 can then be determined using equation (2).

As pointed out earlier, the approach for determining the radius 25 presented above is described in an intuitively accessible way. Since the radius 25 can be determined mathematically based on the above five parameters, the radius 25 can be determined by only using equations without requiring an actual geometrical construction in a coordinate system.

The second method embodiment for determining the radius 25 requires not pivoting of the instrument tip 24 inside the calibration structure 38 and therefore reduces the risk of damages, such as abrasion of the instrument tip 24 and of the calibration structure 38.

The method of the second embodiment may optionally comprise determining the position of the centre point 27. To this end, the method may include determining the second reference point 57 based on the fourth pose and the reference position 53, as described above.

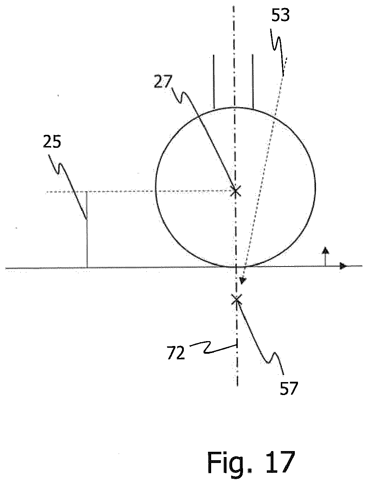

Fig. 17 shows a geometrical relationship between the second reference point 57, the calibration surface 45, the radius 25, and the centre point 27. The method may further comprise determining a reference axis 72 through the second reference point 57 and perpendicular to the calibration surface 45. The reference axis 72 may be defined by any mathematical term that defines a position and orientation of a line, such as a vector sum of a position vector (e.g., the second reference point 57) and an orientation vector (e.g., a vector perpendicular to the calibration surface 45). The method may further comprise determining a position of the centre point 27 of the spherically shaped surface portion 26 relative to the second tracker 22 along the reference axis 72 at a distance of the radius 25 from the reference surface 45 in a direction away from the calibration device 34. When the instrument tracker is in the fourth pose, the spherical surface portion 26 abuts against the calibration surface 45. Therefore, the centre point 27 is located at a distance of the radius 25 away from the calibration surface 25. The centre point 27 can be determined, for example, at an intersection of the reference axis 72 with a plane that is parallel to the calibration surface 45 and spaced at a distance of the radius 25 away from the calibration surface 45. A different way to determine the centre position by adding a vector with the orientation of the reference axis 72 and the combined distance of the radius 25 and the reference distance 71 to a position vector that locates the second reference point 57.

The radius determination approach described above is most accurate for determining the radius 25, and optionally, the position of the centre point 27, when the surgical instrument 20 is oriented identically relative the central axis 42 (when abutted against the calibration structure 38) and the surface normal 49 (when abutted against the calibration surface 45). Different orientations of the surgical instrument 20 in the two abutment positions may result in a less accurate result for the radius 25. Such identical or different orientations are now explained with reference to Figs. 18A to 18F.

Fig. 18A shows an example of the calibration device 34, wherein the central axis 42 of the calibration structure 38 is arranged parallel to the surface normal 49 of the calibration surface 45. A surface angle may define an angular difference between a normal vector of the calibration surface 45 (i.e., the surface normal 49) and a bisector of the opening angle (i.e., the central axis 42). The surface angle of the example shown in Fig. 18A is zero.

Fig. 18B shows an example of the calibration device 34, wherein the central axis 42 of the calibration structure 38 is not arranged parallel to the surface normal 49 of the calibration surface. The surface angle of the example shown in Fig. 18A is 40° and therefore not zero.

Fig. 18C shows the example of the calibration device 34 depicted in Fig. 18A, wherein the orientation of the instrument tip 24, and therefore also for the instrument tracker 22, is identical for the second pose and the fourth pose of the instrument tracker 22. The second pose may define a first calibration orientation of the second tracker 22 and the fourth pose may define a second calibration orientation of the second tracker 22, wherein a reorientation angle defines an angular difference between the first calibration orientation and the second calibration orientation. In the example shown in Fig. 18C, the first calibration orientation and the second calibration orientation are identical. Therefore, the reorientation angle is zero.

Fig. 18D shows the example of the calibration device 34 depicted in Fig. 18B, wherein an orientation of the instrument tip 24 and consequently the instrument tracker 22 does not change relative to the central axis 42 and the surface normal 49. However, as the central axis 42 and the surface normal 49 are not arranged parallel, the first calibration orientation and the second calibration orientation are not identical and the reorientation angle is not zero.

Therefore, the approach suggested herein may further comprise determining a pivot angle based on the reorientation angle and the surface angle. For example, the pivot angle may be defined as a polar angle component in regard to the surface normal 49 of a sum of the reorientation angle and the surface angle. Alternatively, the pivot angle may be defined as a polar angle relative to the surface normal 49 that is required in order to rotate the second calibration orientation relative to the surface normal 49 such that the orientation of the second calibration orientation relative to the surface normal 49 is identical to the orientation of the first calibration orientation relative to the central axis 42. In the example shown in Fig. 18C, the reorientation angle and the surface angle are both zero. Therefore, the sum of the reorientation angle and the surface angle is also zero. Consequently, the pivot angle is also zero.

Another way to determine the pivot angle is to determine a first rotation transformation that rotates the surface normal 49 onto the central axis 42, and to determine a second rotation transformation that rotates the first calibration orientation onto the second orientation calibration. A combined rotation transformation obtained from a product of the first and second rotation transformation is applied to the surface normal 49, wherein the pivot angle can be determined from the resulting rotation of the surface normal 49.

In Fig. 18D, the first calibration orientation and the second calibration orientation are not identical with a reorientation angle of 40° and that is directed clockwise. Similarly, the surface angle is 40° and directed counter-clockwise. Therefore, the reorientation angle and the surface angle cancel each other out, such that the sum of the two angles is zero. As a result, the pivot angle in the example shown in Fig. 18D is also zero.

Fig. 18E shows the example of the calibration device 34 depicted in Fig. 18A, wherein an orientation of the instrument tip 24 and consequently the instrument tracker 22 changes relative to the central axis 42 and the surface normal 49.

Therefore, the reorientation angle is not zero. Since the central axis 42 and the surface normal 49 are parallel, the surface angle is zero. The sum of the surface angle and the reorientation angle is the reorientation angle. In the example shown in Fig. 18E, the reorientation angle only has a polar angle component and no azimuth angle component in regards to the surface normal 49. Therefore, the reorientation angle is also the pivot angle.

Fig. 18F shows the example of the calibration device 34 depicted in Fig. 18B, wherein the reorientation angle and surface angle are both not zero and do not cancel each other out. In the example depicted in Fig. 18F, the sum of the reorientation angle and the surface angle only has a polar angle component and no azimuth angle component in regard to the surface normal 49. Therefore, the pivot angle is the sum of the reorientation angle and the surface angle.

In summary, Figs. 18C and 18D show examples in which the pivot angle is zero and Figs. 18E and 18F show examples in which the pivot angle is not zero. Therefore, the approach described above for determining the radius 25 and the position of the centre point 27 yield accurate results for the examples shown in Figs. 18C and 18D, but would yield results with errors that increase with the pivot angle for the examples shown in Figs. 18E and 18F.

The error can be minimized by instructing the user to orient the surgical instrument 20 in a way that minimizes the pivot angle. Corresponding instructions may be output to a user by the computer system 13 (see Fig. 3), e.g., as part of a radius calibration procedure. Alternatively, or additionally, a guiding device can be provided that guides movement of the surgical instrument 20 such that the pivot angle is minimized (e.g., by a funnel or a structure with an opening).

Furthermore, the second method embodiment may comprise determining the pivot angle and generating output information related to the pivot angle. The output information may include at least one the determined pivot angle and information (e.g., textual information) about the pivot angle exceeding a pivot angle threshold. The pivot angle threshold may be higher than 3°, 5°, 10°, 20°, 30° and 45°. The pivot angle threshold may be 60° or lower, for example 45° or lower. The output information may comprise re-calibration instructions (e.g., a request to repeat the measurement with a smaller pivot angle).

The method may comprise determining the radius 25 also on the basis of an axis pose of an instrument axis relative to the instrument tracker 22 that extends through the centre point 27. The axis pose may be predetermined or determined in a separate step of the method. For example, the axis pose may be determined based on tracking data obtained from the instrument tracker 22 while the surgical instrument 20 is rotated about the instrument axis.

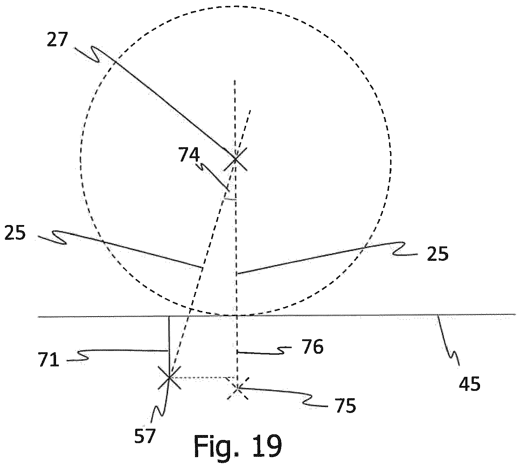

Another approach is for the step of determining the radius 25 to further be based on the pivot angle. For example, equation (2) described above may be modified to further include the pivot angle. Fig. 19 shows the geometrical relationship between the radius 25, the reference distance 71 and the pivot angle 74, which may form the basis for a modified equation.

In case the pivot angle 74 were zero, a hypothetical second reference point 75 would be located along a line through the centre point 27 and perpendicular to the calibration surface 45 and at a target distance 75 equal to the distance between the first reference point 44 and the spherically shaped surface portion 26 when in abutment with the calibration structure 38. However, in the case the pivot angle 74 is not zero, the second reference point 57 is rotated around the centre point 27 by the pivot angle. As a result, the reference distance 71 is shortened. If the shorter reference distance 71 were to be used in equation (2), a radius 25 shorter than the actual radius 25 would be obtained.

Since the position of the centre point 27 and the second reference point 57 relative to the instrument tracker 22 is independent from the pivot angle, the distance between the centre point 27 and the second reference point 57 is unchanged. From the geometrical relationship depicted in Fig. 19 can be obtained the following equation:

$$(r + h)\cos(\alpha) = r + d \qquad \text{equation (3)}$$

with r as the radius 25, d is the determined reference distance 71, h is a hypothetical reference distance 76 between the calibration surface 45 and the hypothetical second reference point 75, and a is the pivot angle 74.

Equation 2 also describes the geometrical relationship when using the hypothetical reference distance 76 for d. For the sake of better legibility, equation (2) can be simplified to

$$r = \frac{h \cdot \sin\left(\frac{\theta}{2}\right)}{1 - \sin\left(\frac{\theta}{2}\right)} = h \cdot c \quad \text{with } c = \frac{\sin\left(\frac{\theta}{2}\right)}{1 - \sin\left(\frac{\theta}{2}\right)}. \qquad \text{equation (4)}$$

Inserting equation (4) into equation (3) results in

$$(hc + h)\cos(\alpha) = hc + d. \qquad \text{equation (5)}$$

Solving equation (5) for h results in

$$h = \frac{d}{(c+1)\cos(\alpha) - c}. \qquad \text{equation (6)}$$

Combining equation (6) with equation (4) results in the following equation for determining the radius 25:

$$r = hc = \frac{dc}{(c+1)\cos(\alpha)-c}.$$    equation (7)

Equation (7) allows determining the radius 25 based on the reference distance 71, the opening angle 40 and the pivot angle 74. These three parameters are known or can be determined as described above.

For larger pivot angles 74 (in particular when the pivot angle is equal to 90° minus half the opening angle 40), the second reference point 57 is located on or close to the reference surface 45. In such a case, the reference distance 71 may shorten to such a small length that a finite resolution of the tracking system 14 may not be able to accurately determine the reference distance 71. Therefore, the method may comprise generating output information about the pivot angle exceeding a pivot angle threshold. The pivot angle threshold may be below 45°, 30°, 25°, or 20°. The output information may comprise instructions to repeat the measurement with a smaller pivot angle.

Since equation (7) allows determining the radius 25 exactly, the position of the centre point 27 can also be determined exactly as will be described below.

As shown in Fig. 17, in the case of a pivot angle that is zero, the centre point 27 is located on a reference axis 72 that is arranged perpendicular to the calibration surface 45. In the case that the pivot angle is not zero, the positions of the centre point 27 and the second reference point 57 relative to the instrument tracker 22 is still unchanged. Therefore, the reference axis 72 can still be defined through the centre point 27 and the second reference point 57. However, the reference axis 72 is then not arranged perpendicular to the calibration surface 45, but rotated by the pivot angle. Such a construction can be used to determine the position of the centre point 27 as described below.

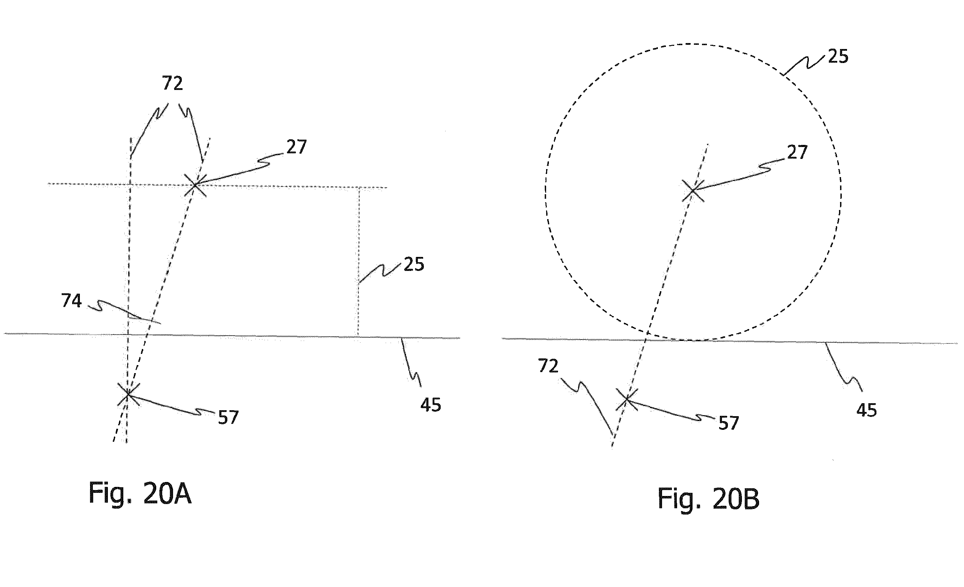

Fig. 20A shows the geometrical relationship of the second reference point 57, the centre point 27, the reference axis 72, and the pivot angle 74. The second method embodiment may comprise defining a reference axis 72 that extends through the second reference point 57 and is initially arranged perpendicular to the calibration surface 45. The method may also comprise rotating the reference axis 72 around the second reference point 27 by the pivot angle 74. The method may further comprise determining the position of the centre point 27 along the reference axis 72 at a distance of the radius 25 from the reference surface 45 in a direction away from the calibration device 34. As can be seen in Fig. 20A, the distance of the radius 25 is not measured along the reference axis 72, but perpendicular to the calibration surface 45. The method may further comprise reconstructing the radius 25 around the centre point 27, as shown in Fig. 20B.

It should be noted that the way to determine the position of the centre point 27 described above is an intuitively accessible way and the position may be alternatively determined in a different way such as an equation-based approach that requires no actual construction of axes and points.

Generally, the method of the second embodiment may comprise determining the position of the centre point 27 based on the third pose, the second predetermined relationship 67, the reference position 53, the fourth pose, the opening angle 40, and the radius 25 and optionally in the case that the central axis 42 and surface normal 49 are not parallel, the orientation of the central axis 42 and the surface normal 49. For example, the method may comprise determining the position of the centre point 27 based on the second reference point 57 (which can be determined based on the fourth pose and the reference position 53), the calibration surface 45 (which can be determined based on the third pose and the second predetermined relationship), the pivot angle (which can be determined based on the second pose and the fourth pose, and, optionally in the case that the central axis 42 and surface normal 49 are not parallel, the orientation of the central axis 42 and the surface normal 49), and the radius 25.

All method embodiments described above allow determining the radius 25 of the spherically shaped surface portion 26 and, optionally, determining the position of the centre point 27. The method embodiments may optionally include further steps that use the radius 25 (and optionally the position of the centre point 27) as will be described below.

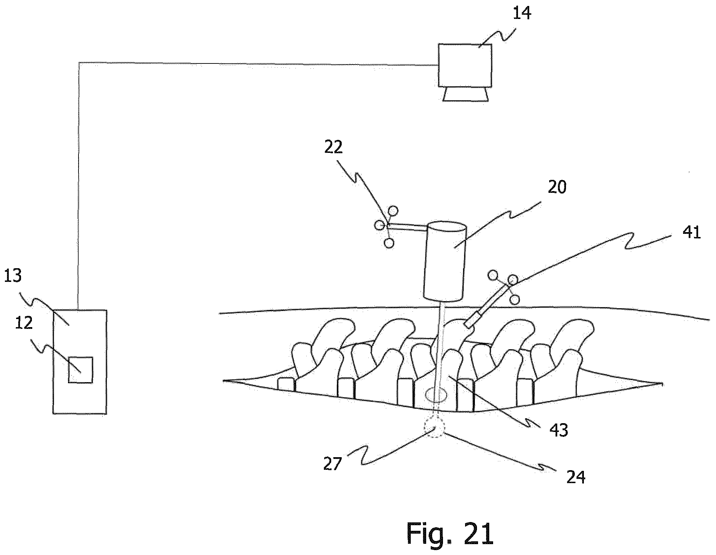

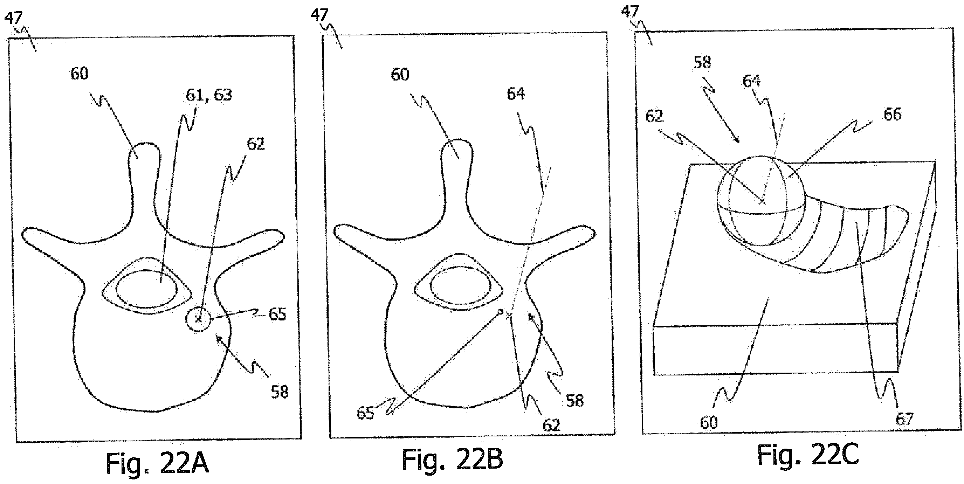

For example, the methods embodiments may further include generating display instructions for displaying a visual representation of the radius 25 in a virtual space (e.g., on a display). As such, the methods may further comprise tracking, during a surgical procedure, a further tracker to register or track an image pose of image data of an object (e.g., a part of a patient anatomy) in the virtual space (e.g., defined by coordinates of the coordinate system 19 of the tracking system 14 or of a coordinate system of image data). Fig. 21 shows an enlarged view of a surgical scenario with the instrument tracker 22 and a patient tracker 41 being tracked by the tracking system 14. The patient tracker 41 shown in Fig. 22 is coupled to an object 43 (in form of a vertebra) to be treated by the surgical instrument 20. As explained above, also the vertebra 43 itself may constitute the patient tracker 41.

The methods may also comprise tracking the instrument tracker 22 to track a position of the centre point 27 of the instrument tip 24 (that has been determined as explained above) in the virtual space based on the position of the centre point 27 relative to the instrument tracker 22. For example, the position of the centre point 27 relative to the instrument tracker 22 may be described by the fourth transformation 35 (see Fig. 10), the reference position 53 (see Fig. 14B) or otherwise. The position of the centre point 27 may then be tracked by tracking the instrument tracker 22 and applying the fourth transformation 35 or the reference position 53 onto the pose of a centre of the instrument tracker 22 thus determined.

As can be seen in Fig. 21, the instrument tip 24 is in this example used to manipulate the vertebra 43 by burring or drilling a hole into the vertebra 43. However, from the outside, the user 18 has only a limited view of the manipulation occurring inside the vertebra 43. The methods thus may further comprise generating display instructions for displaying in the virtual space a visual representation indicative of the radius 25 relative to the image data of the vertebra 43. The display instructions are generated based on the pose of the image data of the vertebra 43 in the virtual space, the position of the centre point 27 in the virtual space, and the radius 25 (possibly after a preceding image data registration as known in the art).

Fig. 22A shows a first example of a virtual space 47 with a visual representation 58 indicative of the radius 25 arranged relative to image data 60 of the vertebra 43 of Fig. 21. The image data 60 may, for example, comprise a point cloud or polygon mesh captured using computer tomography, magnetic resonance imaging, or any other medical imaging procedure. The image data 60 can be arranged in the virtual space 47 based on the image pose of the image data, which was determined based on tracking the tracker 41 and a preceding registration. The visual representation 58 can be arranged and updated in the virtual space 47 based on the tracked instrument tracker 22. Consequently, the image data 60 and the visual representation can be arranged relative to each other in the virtual space 47. The virtual space 47 may be defined, for example, by a coordinate system of the tracking system 14 or a coordinate system of the image data 60.

The exemplary visual representation 58 shown in Fig. 22A comprises a virtual centre point 62 at the position of the centre point 27 and a circle 65 around the centre point 27 with the radius 25 as determined during the calibration procedure. In some variants, the radius visualization may comprise an icon selected based on the determined radius 25 (e.g., an icon of the instrument tip 24). Of course, in other examples, only the circle 65 or only a portion thereof may be visualized. Needless to say that the image data 60 and any radius indication may need to be converted to a common scale for visualization.

Through the vertebra extends a spinal cord 61 that is not to be touched by the instrument tip 24. Since the visual representation 58 includes an indication of the radius 25, the user 18 can see when the instrument tip 24 comes too close to the spinal cord 61 and is guided to adapt instrument movement accordingly. As the radius 25 has exactly been determined earlier, the corresponding visual guidance is very precise.

Optionally, the method embodiments may include generating display instructions for displaying a non-target region 63 in the image data 60 for which contact with the instrument 20 is to be avoided. In the example shown in Fig. 22A, the non-target region 63 is identical with the region of the spinal cord 61. Alternatively, the non-target region may be larger than the spinal cord 61 or comprise a threshold region around the region of the spinal cord 61. The method embodiments may comprise tracking the position of the radius 25 of the instrument tip 24 and outputting a warning when the tracked instrument tip 24 comes in contact with the non-target region 63 and/or the threshold region. The warning may be at least one of an acoustic, optical, and haptic warning.

Fig. 22B shows a second example of a virtual space 47 with a visual representation 58 indicative of the radius 25 arranged relative to image data 60 of the vertebra (see Fig. 21). The visual representation 58 comprises the position of the centre point 27 and a radius indication 65 in form of a point arranged at a distance of the radius 25 from the centre point 27. The radius indication 65 covers less area of the image data 60 of the vertebra, giving the user 18 a better view of the image data 60. The radius indication 65 may be stationary (e.g., relative to an instrument axis 64 or the virtual space 47). Alternatively, the method may comprise determining a movement direction of the instrument tip 24 and generating display instructions for which the radius indication 65 moves towards the determined movement direction of the instrument tip 24. The radius indication 65 may be larger than a point.

As shown Fig. 22B, the visual representation 58 may further be indicative of the instrument axis 64. A pose of the instrument axis 64 may be determined on the basis of tracking the instrument tracker 22 while the surgical instrument 20 is rotated about the instrument axis 64. Alternatively, or additionally, the instrument axis 64 may be determined based on tracking the instrument tracker 22 and having access to a known predetermined spatial relationship between the instrument tracker 22 and the instrument axis 64. The predetermined spatial relationship may be provided in form of a transformation that maps the pose of the instrument tracker 22 onto the pose of the instrument axis 64. The pose of the instrument axis 64 can therefore be determined by tracking the instrument tracker 22 in order to obtain the pose of the instrument tracker 22 and applying the transformation onto the pose of the instrument tracker 22.

Fig. 22C shows a third example of a virtual space 47 with a visual representation 58 of the instrument 20 arranged relative to image data 60 of an object in form of a bone surface. The visual representation 58 comprises the position of the centre point 27 (optional) and a sphere 66 around the centre point 27 with the determined radius 25. Displaying the radius 25 in form of a three dimensional sphere 66 may be used when the image data 60 or a tissue manipulation progress is also displayed in a three dimensional space. Fig. 13C shows a tissue manipulation progress 67 determined for a path of the visual representation 58. The tissue manipulation progress 67 may be determined based on the determined radius 25, a tracking of the instrument tracker 22, and the image data 60.

The method embodiments may further comprise outputting, on a display, the determined radius 25 of the spherically shaped surface portion 26 of the instrument tip 24 as a numerical value. The user 18 may be requested to confirm the numerical value prior to the surgical procedure.

The method embodiments may further comprise displaying, on a display (e.g., of the tracking system 14 or surgical navigation system 10), a visual representation of the instrument 20 arranged relative to the image data of the object. The visual representation of the instrument 20 may take the form of an icon.

The method embodiments may further comprise determining from a pre-determined list of radii a subset of radii that meet a similarity criterion with respect to the determined radius 25. The pre-determined list of radii may comprise the radii of various instruments tips 24 the user may selectively attach to the instrument 20 or of various instruments 20 with different instrument tips 24. The instrument tips 24 may differ in the tip radii and in the tip configurations (see Figs. 4A to 4C). The same radius may be associated with different tip configurations, and the same tip configuration may be associated with different radii. The list may have a length of 10, 50 or more list entries.

The similarity criterion may comprise being a radius from the subset that differs by not more than a difference threshold from the determined radius 25. For example, the similarity criterion may comprise being a radius that differs by not more than 1.0mm from the determined radius 25. Given a scenario where the determined radius 25 were to be determined as 5.3mm, the subset would include radii from an interval of 4.3mm and 6.3mm. The difference threshold may define any other distance such as 0.1mm, 0.3mm, 0.5mm, or 2.0mm.

Additionally, or alternatively, the similarity criterion may comprise a number of radii that are closest to the determined radius 25. The number of radii closest to the determined radius 25 may be any number larger than one, such as two, three, four, five, or more. For example, if the number of radii closest to the determined radius 25 is five, a subset of radii may be determined that comprises the five radii that are the closest to the determined radius 25.

The similarity criterion may combine multiple criteria. For example, in a first step an intermediate set of radii that differ by not more than a difference threshold is determined. In a subsequent step, of the intermediate set of radii a subset of a number of radii that are closest to the determined radius 25 is determined.

The similarity criterion may vary with the pivot angle. For example, the difference threshold and/or number or radii that are closest to the determined radius 25 may increase with the pivot angle 74.

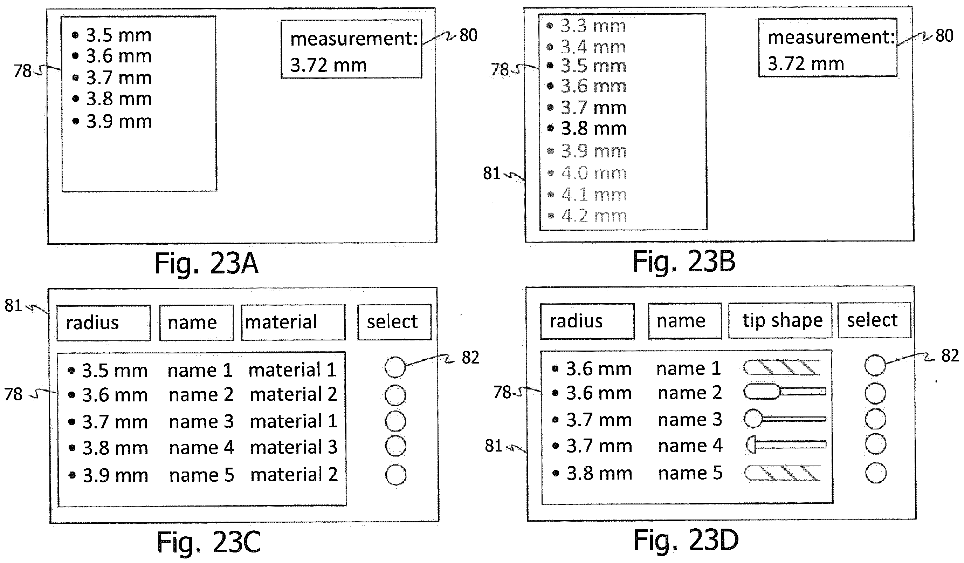

Fig. 23A shows a subset of radii 78 that meet a similarity criterion with a difference threshold. Fig. 23A may show a visual representation of data of the device 12 that comprises the subset of radii 78 or may alternatively show an output on a display for a user about the determined subset of radii 78. In the example shown in Fig. 23A, a radius 25 of 3.72mm has been determined, which may optionally be display to the user in form of a numerical radius output 80.

The subset of radii 78 may be displayed isolated from the rest of the predetermined list of radii as shown in Fig. 23A. Alternatively, at least a part of the pre-determined list may be displayed, wherein the subset of radii 78 is highlighted. Fig. 23B shows a display output 81 displaying a subset of radii 78 highlighted within the pre-determined list of radii.

The method may further comprise generating an output for a user selection or confirmation, wherein the output comprises at least one of information about the subset of radii 78 and information about instrument tips 24 associated with the subset of radii 78. The information about instrument tips 24 may comprise at least one of tip name, tip material, tip manufacturer, instrument tip use, previous use of an instrument tip, and compatibility with a current or planned surgical procedure. Fig. 23C shows a display output 81 displaying a subset of radii 78 with information about the subset of radii 78 and information about instrument tips 24 associated with the subset of radii 78. In the example shown in Fig. 23C, the information about instrument tips 24 comprises a name of the instrument (e.g., manufacturer name or a label of the instrument tip 24) and material (e.g., martensitic, austenitic or ferritic steel), but may alternatively, or additionally, comprise any other information as described above. The display output 81 further comprises an input region 82 that allows the user to select or confirm an instrument tip 24 from the subset of radii 78. The input region 82 may overlap or coincide with any information shown of the corresponding instrument tip 24 (e.g., the user may select an instrument tip 24 by clicking on the name or the radius of the instrument tip 24).

The list of radii may comprise multiple instrument tips 24 with a same radius that differ in a different quality such as material or manufacturer. Determining the radius 25 of the instrument tip 24 may not allow distinguishing between instrument tips 24 with the same radius. The subset of radii may inform the user that not a single instrument tip 24 is assigned to the determined radius 25. In such a case, user selection or confirmation of the instrument tip allows determining the correct instrument tip 24.

Fig. 23D shows a display output displaying a subset of radii 78 with information about the subset of radii 78, wherein some instrument tips 24 have the same radius 25 (e.g., two instrument tips 24 with a radius of 3.6 mm), but differ in the tip shape (see Figs. 4A to 4C). The display output 81 further comprises an input region 82 that allows the user to select or confirm an instrument tip 24 from the subset of radii 78.

In the case that determining the radius 25 contains errors, the determined radius 25 may not properly reflect the actual radius. The list of radii indicates likely candidates of the actual radius. User selection or confirmation may therefore provide the required information for correctly identifying the actual radius (as used, later on, for example in a navigation procedure as illustrated in Figs. 22A to 22C).

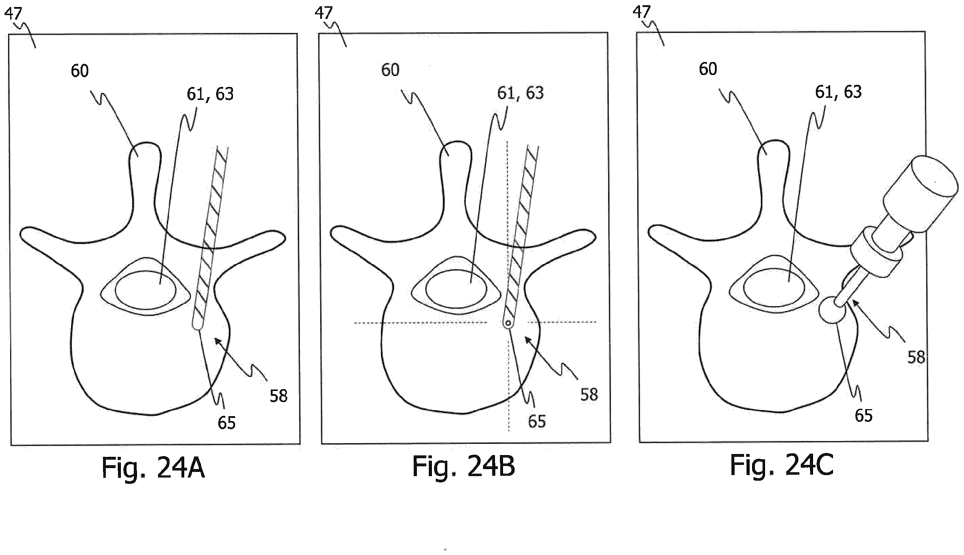

The method embodiments may comprise determining the instrument tip 24 based on at least one of the determined radius 25, a confirmation of the user, and a selection of the user. The visual representation 58 indicative of the radius 25 may comprise a two-dimensional or three-dimensional instrument model icon of the determined instrument tip 24. Fig. 24A shows a fourth example of a virtual space 47 with an instrument model icon as a visual representation 58 indicative of the radius 25 arranged relative to image data 60 of the vertebra 43. The visual representation 58 is a two-dimensional instrument model icon of the determined instrument 24. An end portion of the instrument model comprises a radius indication 65.

Fig. 24B shows a fifth example of a virtual space 47 with an instrument model icon as a visual representation 58. The fifth example essentially differs from the fourth example in that the visual representation 58 further indicates a position of the centre point 27. The centre point 27 may alternatively or additionally be indicated by a reticle such as a crosshair, for example as shown in Fig. 24B.

Fig. 24C shows a sixth example of a virtual space 47, wherein the visual representation comprises a three-dimensional model of the surgical instrument 20 (e.g., in the form of an icon). The three-dimensional model can be rotated and therefore is able to fully reflect an orientation of the surgical instrument 20. A three-dimensional model is in particular suitable for navigating a surgical instrument 20 relative to three dimensional image data 60.

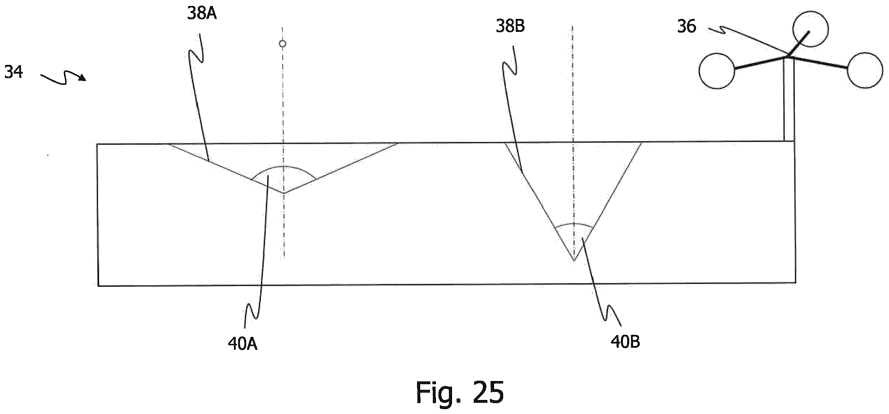

The calibration devices 34 described herein may comprise a plurality of calibration structures 38. Such a calibration device 34 allows simultaneous calibration for a plurality of surgical instruments 20. Additionally, at least two of the calibration structures 38 may have a different opening angle 40. Fig. 25 shows a calibration device 34 with a first calibration structure 38A and a second calibration structure 38B. The first calibration structure 38A has a first opening angle 40A and the second calibration structure 38B has a second opening angle 40B, wherein the first opening angle 40A is larger than the second opening angle 40B. For the calibration structures 38A, 38B, the central axes are arranged in parallel. Alternatively, the central axes of the calibration structures 38A, 38B may be arranged in a non-parallel way (e.g., 45°, 90° or 135°).

The first opening angle 40A results in a larger diameter of the opening of the first calibration structure 38A and consequently allows insertion of an instrument tip 24 with a larger radius 25 compared to the second calibration structure 38B. On the other hand, the comparably smaller second opening angle 40b of the second calibration structure 38B translates a difference of the radius 25 of the instrument tip 24 to a larger difference of the centre distance 56 between the position of the apex 44 and the position of the centre point 27 and the spherically shaped surface portion 26. Since the larger difference of the centre distance 56 can be better resolved with a limited spatial accuracy of the surgical navigation system 10, the second calibration structure 38B provides a more accurate radius determination. The calibration device 34 can therefore be used in combination with instrument tips 24 that have a much larger or smaller radius. As a result, a calibration device 34 with different opening angles 40A, 40B increases a range of usable tip radii 25 and consequently increases the flexibility of instrument selection for the user 18.

However, since the opening angle 40A, 40B is required for determining the radius 25, as an intermediate step, the method may include a step for determining the position and, optionally in case the opening angles 40A, 40B differ, the opening angle 40A, 40B of the calibration structure 38A, 38B in which the instrument tip is arranged by identifying the calibration structure 38A, 38B based on the position of the centre point 27.

The calibration structure 38A, 38B may be identified based the position of the centre point 27 and on the predetermined first relationship alone, e.g., based on a vicinity of the centre point 27 relative to the apex 44. Additional information may be provided, such as coordinates of a volume inside the calibration structure 38A, 38B. The calibration structure 38A, 38B may then be identified based on whether or not the position of the centre point 27 is inside the volume of either one of the calibration structures 38A, 38B.

In case a calibration structure 38A, 38B cannot be identified (e.g., the distance to the apex 44 exceeds a predetermined threshold, or the position of the centre point 27 is outside a volume of both calibration structures 38A, 38B), the method may comprise outputting an error message to the user 18.

Determining the radius 25 of the spherically shaped surface portion 26 gives access to valuable information for the user 18. The user 18 can obtain or verify the radius 25 right before moving the surgical instrument 20 in a surgical procedure, eliminating the risk of a mix up of an instrument tip 24 with a different radius and eliminating the need of managing labels or interaction with a user interface. The user 18 can consequently work more time efficiently and with a lower probability of causing harm to the patient. The surgical navigation system 10 gains the ability to improve accuracy of tracking the instrument tip 24, guiding the user 18, and planning tissue manipulation carried out with the instrument tip 24. The surgical procedure can therefore be carried out more efficiently and safely.

The tracking system 14 need not be attached to a surgical object such as the surgical instrument 20, the calibration device 34, or the patient. Instead, the tracking system 14 may be provided spatially separate from the surgical objects, overlooking the entire surgical environment 16. Such a position enables the tracking system 14 to track a plurality of surgical objects inside the surgical environment 16 and not only the surgical object to which the tracking system 14 is attached. When tracking a plurality of surgical objects, the surgical navigation system 10 is able to apply the determined radius 25 to interactions between the surgical instrument 20 and other tracked surgical objects. The surgical navigation system 10 tracks the surgical instrument 20 and the patient. Once the radius 25 has been determined, the device 12 can guide the user more accurately. The accuracy of the surgical procedure is therefore improved.

It is to be noted that the various visualization approaches taught herein (see, e.g., Figs. 23 and 24) are based on a radius determination (also called radius calibration herein) of a spherically shaped portion of an instrument tip. In the present disclosure, various radius determination techniques have been presented. It is to be understood that the visualization approaches taught herein could also be implemented using any other radius determination technique, including radius determination by user input or user selection, radius measurement using other calibration structures, and so on. As such, embodiments of the visualization approaches taught herein could in general be based on a pre-determined radius of a spherically shaped portion of an instrument tip, no matter how the radius is input, measured, calibrated or determined otherwise.

The features described in relation to the exemplary embodiments shown in the drawings can be readily combined to result in different embodiments. It is apparent, therefore, that the present disclosure may be varied in many ways. Such variations are not to be regarded as a departure from the scope of the invention as defined by the claims appended hereto.

## Claims

1. A computer-implemented method (100) for determining a radius (25) of a spherically shaped surface portion (26) of a tip (24) of an instrument (20), wherein a calibration device (34) is provided that comprises a flat calibration surface (45) and a calibration structure (38), wherein the calibration structure defines an opening angle (40) and a real or imaginary first reference point (44) relative to which the opening angle (40) is defined, wherein the calibration device (34) comprises a first tracker (36) trackable by a tracking system (14) and arranged in a first predetermined relationship (54) relative to the first reference point (44) and in a second predetermined relationship relative to the calibration surface (45), and wherein the instrument (20) comprises a second tracker (22) trackable by the tracking system (14), the method (100) comprising the following steps performed by a computer system (13):

   determining (202) a first pose of the first tracker (36) and a second pose of the second tracker (22) while the spherically shaped surface portion (26) is in abutment with the calibration structure (38);
   determining (204) a reference position (53) of the first reference point (44) relative to the second tracker (22) when the first tracker (36) is in the first pose and the second tracker (22) is in the second pose based on the first pose, the second pose, and the first predetermined relationship (54);
   determining (206) a third pose of the first tracker (36) and a fourth pose of the second tracker (22) while the spherically shaped surface portion (26) is in abutment with the calibration surface (45); and
   determining (208) the radius (25) of the spherically shaped surface portion (26) based on the third pose, the second predetermined relationship, the reference position (53), the fourth pose, and the opening angle (40).

2. The computer-implemented method (100) according to claim 1, wherein determining the radius (25) of the spherically shaped surface portion (26) comprises

   determining a second reference point (57) based on the fourth pose and the reference position (53);
   determining a reference distance (71) defining a distance between the second reference point (57) and the calibration surface (45) based on the second reference point (57), the third pose, and the second predetermined relationship; and
   determining the radius (25) based on the reference distance (71) and a trigonometric function of the opening angle (40).

3. The computer-implemented method according to claim 1 or 2, wherein

   the second pose defines a first calibration orientation of the second tracker (22) and the fourth pose defines a second calibration orientation of the second tracker (22), wherein a reorientation angle defines an angular difference between the first calibration orientation and the second calibration orientation, wherein a surface angle defines an angular difference between a normal vector (49) of the calibration surface (45) and a bisector (42) of the opening angle (40), the method further comprising
   determining a pivot angle (74) based on the reorientation angle and the surface angle.

4. The computer-implemented method (100) according to claim 3, wherein determining (106) the radius (25) of the spherically shaped surface portion (26) is further based on the pivot angle (74).

5. The computer-implemented method (100) according to any of the preceding claims, further comprising

   determining a second reference point (57) based on the fourth pose and the reference position (53);
   determining a reference axis (72) through the second reference point (57) and perpendicular to the calibration surface (45); and
   determining a position of a centre point (27) of the spherically shaped surface portion (26) relative to the second tracker (22) along the reference axis (72) at a distance of the radius (25) from the reference surface in a direction away from the calibration device (34).

6. The computer-implemented method (100) according to claim 5 in combination with any of claim 3 or 4, comprising rotating the reference axis (72) around the second reference point (57) by the pivot angle (74) before determining the position of the centre point (27) along the reference axis (72).

7. A computer-implemented method (100) for determining a radius (25) of a spherically shaped surface portion (26) of a tip (24) of an instrument (20), wherein the spherically shaped surface portion (26) has a centre point (27), wherein a calibration device (34) is provided that comprises a calibration structure (38) defining an opening angle (40) and that is configured to cooperate with the spherically shaped surface portion (26), wherein the spherically shaped surface portion (26) is configured to slide along the calibration structure (38) in a process of guiding a tilting movement of the instrument tip (24) around the centre point (27), wherein the calibration device (34) comprises a first tracker (36) trackable by a tracking system (14) and arranged in a predetermined relationship relative to the calibration structure (38), and wherein the instrument (20) comprises a second tracker (22) trackable by the tracking system (14), the method (100) comprising the following steps performed by a computer system (13):

   determining (102) a first pose of the first tracker (36);
   determining (104) positional data of the second tracker (22) for different tilting positions of the instrument (20) while the spherically shaped surface portion (26) is in abutment with the calibration structure (38);
   determining (106) a position of the centre point (27) relative to the second tracker (22) based on the positional data; and
   determining (108) the radius (25) of the spherically shaped surface portion (26) based on the position of the centre point (27), the first pose, the predetermined relationship, and the opening angle (40).

8. The computer-implemented method (100) according to claim 7, comprising
   determining the radius (25) based on a centre distance (56) between the position of the centre point (27), a real or imaginary point (44) relative to which the opening angle (40) is defined and a trigonometric function of the opening angle (40).

9. The computer-implemented method (100) according to claim 7 or 8, wherein the positional data comprise at least one of

   - at least two different tilt poses of the second tracker (22) for the different tilting positions, and
   - at least four different positions of the second tracker (22) for the different tilting positions.

10. The computer-implemented method (100) according to any of the preceding claims, wherein
    the calibration structure (38) has a shape of a cone or a pyramid, or portion thereof, defining the opening angle (40).

11. The computer-implemented method (100) according to any of the preceding claims, comprising
    determining from a pre-determined list of radii a subset of radii that meet a similarity criterion with respect to the determined radius.

12. The computer-implemented method according to claim 11, comprising
    generating an output for a user selection or confirmation, wherein the output comprises at least one of information about the subset of radii and information about instrument tips (24) associated with the subset of radii.

13. The computer-implemented method according to any of the claims 5, 6 or 7 to 9, or a claim depending therefrom, comprising

    tracking (108) a third tracker (41) of an object (43) and trackable by the tracking system (14) to track an object pose of image data (60) of the object (43) in a virtual space (47);

tracking (110) the second tracker (22) to track a position of the centre point (27) in the virtual space (47) based on the position of the centre point (27) relative to the second tracker (22); and

generating (112) first display instructions for displaying in the virtual space (47), based on the object pose of the image data (60) of the object (43) in the virtual space (47), the position of the centre point (27) in the virtual space (47) and the radius (25), a visual representation (58) indicative of the radius (25) relative to the image data (60) of the object (43).

14. The computer-implemented method (100) according to claim 13, comprising

generating region display instructions for displaying a non-target region (63) in the image data (60) for which contact with the instrument (20) is to be avoided.

15. The computer-implemented method (100) according to claim 13 or 14, comprising

generating progress display instructions for displaying a progress (67) of object manipulation based on a path of the instrument tip (24).

16. The computer-implemented method (100) according to any of claims 13 to 15, comprising

displaying, on a display, a visual representation (58) of at least a portion of the instrument (20) relative to the image data (60) of the object (43).

17. The computer-implemented method (100) according to any of the claims 13 to 16, wherein

the visual representation (58) comprises an icon representative of at least a part of a circle (65) or a sphere (66) with the radius (25) of the spherically shaped surface portion (26).

18. A computer program product, comprising instructions that, when executed on at least one processor, cause the at least one processor to carry out any of the methods of claims 1 to 17.

19. A device (12) for determining a radius (25) of a spherically shaped surface portion (26) of a tip (24) of an instrument (20), wherein a calibration device (34) is provided that comprises a flat calibration surface (45) and a calibration structure (38), wherein the calibration structure (38) defines an opening angle (40) and a real or imaginary first reference point (44) relative to which the opening angle (40) is defined, wherein the calibration device (34) comprises a first tracker (36) trackable by a tracking system (14) and arranged in a first predetermined relationship (54) relative to the first reference point (44) and in a second predetermined relationship relative to the calibration surface (45), and wherein the instrument (20) comprises a second tracker (22) trackable by the tracking system (14), the device configured to

determine a first pose of the first tracker (36) and a second pose of the second tracker (22) while the spherically shaped surface portion (26) is in abutment with the calibration structure (38);

determine a reference position (53) of the first reference point (44) relative to the second tracker (22) when the first tracker (36) is in the first pose and the second tracker (22) is in the second pose based on the first pose, the second pose, and the first predetermined relationship (54);

determine a third pose of the first tracker (36) and a fourth pose of the second tracker (22) while the spherically shaped surface portion (26) is in abutment with the calibration surface (45); and

determine the radius (25) of the spherically shaped surface portion (26) based on the third pose, the second predetermined relationship, the reference position (53), the fourth pose, and the opening angle (40).

20. The device (12) according to claim 19, configured to perform any of the method steps according to claims 2 to 6 and 10 to 17.

21. A device (12) for determining a radius (25) of a spherically shaped surface portion (26) of a tip (24) of an instrument (20), wherein the spherically shaped surface portion (26) has a centre point (27), wherein a calibration device (34) is provided that comprises a calibration structure (38) defining an opening angle (40) and that is configured to cooperate with the spherically shaped surface portion (26), wherein the spherically shaped surface portion (26) is configured to slide along the calibration structure (38) in a process of guiding a tilting movement of the instrument tip (24) around the centre point (27), wherein the calibration device (34) comprises a first tracker (36) trackable by a tracking system (14) and arranged in a predetermined relationship relative to the calibration structure (38), and wherein the instrument (20) comprises a second tracker (22) trackable by the tracking system (14), the device configured to

determine a first pose of the first tracker (36);
determine positional data of the second tracker (22) for different tilting positions of the instrument (20) while the spherically shaped surface portion (26) is in abutment with the calibration structure (38);
determine a position of the centre point (27) relative to the second tracker (22) based on the positional data; and determine the radius (25) of the spherically shaped surface portion (26) based on the position of the centre point (27), the first pose, the predetermined relationship, and the opening angle (40).

22. The device (12) according to claim 21, configured to perform any of the method steps according to claims 8 to 17.

**Patentansprüche**

1. Computerimplementiertes Verfahren (100) zum Bestimmen eines Radius (25) eines sphärisch geformten Oberflächenabschnitts (26) einer Spitze (24) eines Instruments (20), wobei eine Kalibrierungsvorrichtung (34) bereitgestellt ist, die eine ebene Kalibrierungsoberfläche (45) und eine Kalibrierungsstruktur (38) umfasst, wobei die Kalibrierungsstruktur einen Öffnungswinkel (40) definiert und einen realen oder imaginären ersten Referenzpunkt (44), relativ zu dem der Öffnungswinkel (40) definiert ist, wobei die Kalibrierungsvorrichtung (34) einen ersten Verfolger (36) umfasst, der durch ein Verfolgungssystem (14) verfolgbar ist und in einer ersten vorbestimmten Beziehung (54) relativ zu dem ersten Referenzpunkt (44) und in einer zweiten vorbestimmten Beziehung relativ zu der Kalibrierungsoberfläche (45) angeordnet ist, und wobei das Instrument (20) einen zweiten Verfolger (22) umfasst, der durch das Verfolgungssystem (14) verfolgbar ist, wobei das Verfahren (100) die folgenden Schritte umfasst, die durch ein Computersystem (13) durchgeführt werden:

   Bestimmen (202) einer ersten Pose des ersten Verfolgers (36) und einer zweiten Pose des zweiten Verfolgers (22), während der sphärisch geformte Oberflächenabschnitt (26) an der Kalibrierungsstruktur (38) anliegt;
   Bestimmen (204) einer Referenzposition (53) des ersten Referenzpunktes (44) relativ zu dem zweiten Verfolger (22) auf der Grundlage der ersten Pose, der zweiten Pose und der ersten vorbestimmten Beziehung (54), wenn der erste Verfolger (36) in der ersten Pose und der zweite Verfolger (22) in der zweiten Pose ist;
   Bestimmen (206) einer dritten Pose des ersten Verfolgers (36) und einer vierten Pose des zweiten Verfolgers (22), während der sphärisch geformte Oberflächenabschnitt (26) an der Kalibrierungsoberfläche (45) anliegt; und
   Bestimmen (208) des Radius (25) des sphärisch geformten Oberflächenabschnitts (26) auf der Grundlage der dritten Pose, der zweiten vorbestimmten Beziehung, der Referenzposition (53), der vierten Pose und des Öffnungswinkels (40).

2. Computerimplementiertes Verfahren (100) nach Anspruch 1, wobei die Bestimmung des Radius (25) des sphärisch geformten Oberflächenabschnitts (26) umfasst:

   Bestimmen eines zweiten Referenzpunktes (57) basierend auf der vierten Pose und der Referenzposition (53);
   Bestimmen eines Referenzabstandes (71), der einen Abstand zwischen dem zweiten Referenzpunkt (57) und der Kalibrierungsoberfläche (45) definiert, auf der Grundlage des zweiten Referenzpunktes (57), der dritten Pose und der zweiten vorbestimmten Beziehung; und
   Bestimmen des Radius (25) auf der Grundlage des Referenzabstands (71) und einer trigonometrischen Funktion des Öffnungswinkels (40).

3. Computerimplementiertes Verfahren nach Anspruch 1 oder 2, wobei die zweite Pose eine erste Kalibrierungsorientierung des zweiten Verfolgers (22) definiert und die vierte Pose eine zweite Kalibrierungsorientierung des zweiten Verfolgers (22) definiert, wobei ein Umorientierungswinkel eine Winkeldifferenz zwischen der ersten Kalibrierungsorientierung und der zweiten Kalibrierungsorientierung definiert, wobei ein Oberflächenwinkel eine Winkeldifferenz zwischen einem Normalenvektor (49) der Kalibrierungsoberfläche (45) und einer Winkelhalbierenden (42) des Öffnungswinkels (40) definiert, wobei das Verfahren ferner umfasst:
   Bestimmen eines Schwenkwinkels (74) auf der Grundlage des Umorientierungswinkels und des Oberflächenwinkels.

4. Computerimplementiertes Verfahren (100) nach Anspruch 3, wobei das Bestimmen (106) des Radius (25) des sphärisch geformten Oberflächenabschnitts (26) ferner auf der Grundlage des Schwenkwinkels (74) erfolgt.

5. Computerimplementiertes Verfahren (100) nach einem der vorangehenden Ansprüche, ferner umfassend:

   Bestimmen eines zweiten Referenzpunktes (57) auf der Grundlage der vierten Pose und der Referenzposition (53);

Bestimmen einer Referenzachse (72) durch den zweiten Referenzpunkt (57) und senkrecht zu der Kalibrierungsoberfläche (45); und

Bestimmen einer Position eines Mittelpunkts (27) des sphärisch geformten Oberflächenabschnitts (26) relativ zu dem zweiten Verfolger (22) entlang der Referenzachse (72) in einem Abstand des Radius (25) von der Referenzoberfläche in einer Richtung weg von der Kalibrierungsvorrichtung (34).

6. Computerimplementiertes Verfahren (100) nach Anspruch 5 in Kombination mit einem der Ansprüche 3 oder 4, umfassend

Drehen der Referenzachse (72) um den zweiten Referenzpunkt (57) in Höhe des Schwenkwinkels (74) vor der Bestimmung der Position des Mittelpunkts (27) entlang der Referenzachse (72).

7. Computerimplementiertes Verfahren (100) zum Bestimmen eines Radius (25) eines sphärisch geformten Oberflächenabschnitts (26) einer Spitze (24) eines Instruments (20), wobei der sphärisch geformte Oberflächenabschnitt (26) einen Mittelpunkt (27) aufweist, wobei eine Kalibrierungsvorrichtung (34) bereitgestellt ist, die eine Kalibrierungsstruktur (38) umfasst, die einen Öffnungswinkel (40) definiert und die ausgebildet ist, um mit dem sphärisch geformten Oberflächenabschnitt (26) zusammenzuwirken, wobei der sphärisch geformte Oberflächenabschnitt (26) ausgebildet ist, um entlang der Kalibrierungsstruktur (38) in einem Prozess des Führens einer Kippbewegung der Instrumentenspitze (24) um den Mittelpunkt (27) zu gleiten, wobei die Kalibrierungsvorrichtung (34) einen ersten Verfolger (36) umfasst, der durch ein Verfolgungssystem (14) verfolgbar und in einer vorbestimmten Beziehung relativ zu der Kalibrierungsstruktur (38) angeordnet ist, und wobei das Instrument (20) einen zweiten Verfolger (22) umfasst, der durch das Verfolgungssystem (14) verfolgbar ist, wobei das Verfahren (100) die folgenden Schritte umfasst, die durch ein Computersystem (13) durchgeführt werden:

Bestimmen (102) einer ersten Pose des ersten Verfolgers (36);

Bestimmen (104) von Positionsdaten des zweiten Verfolgers (22) für verschiedene Neigungspositionen des Instruments (20), während der sphärisch geformte Oberflächenabschnitt (26) an der Kalibrierungsstruktur (38) anliegt;

Bestimmen (106) einer Position des Mittelpunkts (27) relativ zu dem zweiten Verfolger (22) auf der Grundlage der Positionsdaten; und

Bestimmen (108) des Radius (25) des sphärisch geformten Oberflächenabschnitts (26) auf der Grundlage der Position des Mittelpunkts (27), der ersten Pose, der vorbestimmten Beziehung und des Öffnungswinkels (40).

8. Computerimplementiertes Verfahren (100) nach Anspruch 7, umfassend:

Bestimmen des Radius (25) auf der Grundlage eines Mittenabstands (56) zwischen der Position des Mittelpunkts (27) und einem realen oder imaginären Punkt (44), relativ zu dem der Öffnungswinkel (40) definiert ist, und einer trigonometrischen Funktion des Öffnungswinkels (40).

9. Computerimplementiertes Verfahren (100) nach Anspruch 7 oder 8, wobei die Positionsdaten

- mindestens zwei verschiedene Neigungsposen des zweiten Verfolgers (22) für die verschiedenen Neigungspositionen, und/oder
- mindestens vier verschiedene Positionen des zweiten Verfolgers (22) für die verschiedenen Neigungspositionen umfassen.

10. Computerimplementiertes Verfahren (100) nach einem der vorangehenden Ansprüche, wobei die Kalibrierungsstruktur (38) die Form eines Kegels oder einer Pyramide oder eines Abschnitts davon hat, die den Öffnungswinkel (40) definiert.

11. Computerimplementiertes Verfahren (100) nach einem der vorangehenden Ansprüche, umfassend:

Bestimmen einer Teilmenge von Radien aus einer vorbestimmten Liste von Radien, die ein Ähnlichkeitskriterium in Bezug auf den bestimmten Radius erfüllen.

12. Computerimplementierte Verfahren nach Anspruch 11, umfassend:

Erzeugen einer Ausgabe für eine Benutzerauswahl oder -bestätigung, wobei die Ausgabe Informationen über die Untergruppe von Radien und/oder Informationen über Instrumentenspitzen (24), die der Untergruppe von Radien zugeordnet sind, umfasst.

13. Computerimplementiertes Verfahren nach einem der Ansprüche 5, 6 oder 7 bis 9 oder einem davon abhängigen Anspruch, umfassend:

Verfolgen (108) eines dritten Verfolgers (41) eines Objekts (43), der durch das Verfolgungssystem (14) verfolgbar ist, um eine Objekt-Pose von Bilddaten (60) des Objekts (43) in einem virtuellen Raum (47) zu verfolgen; Verfolgen (110) des zweiten Verfolgers (22), um eine Position des Mittelpunkts (27) in dem virtuellen Raum (47) basierend auf der Position des Mittelpunkts (27) relativ zu dem zweiten Verfolger (22) zu verfolgen; und Erzeugen (112) von ersten Anzeigeanweisungen, um auf der Grundlage der Objekt-Pose der Bilddaten (60) des Objekts (43) in dem virtuellen Raum (47), der Position des Mittelpunkts (27) in dem virtuellen Raum (47) und des Radius (25) eine visuelle Darstellung (58), die den Radius (25) relativ zu den Bilddaten (60) des Objekts (43) angibt, in dem virtuellen Raum (47) anzuzeigen.

14. Computerimplementiertes Verfahren (100) nach Anspruch 13, umfassend:
Erzeugen von Bereichsanzeigeanweisungen, um einen Nicht-Zielbereich (63) in den Bilddaten (60) anzuzeigen, für den ein Kontakt mit dem Instrument (20) zu vermeiden ist.

15. Computerimplementiertes Verfahren (100) nach Anspruch 13 oder 14, umfassend:
Erzeugen von Fortschrittsanzeige-Instruktionen, um einen Fortschritt (67) der Objektmanipulation basierend auf einem Pfad der Instrumentenspitze (24) anzuzeigen.

16. Computerimplementiertes Verfahren (100) nach einem der Ansprüche 13 bis 15, umfassend:
Anzeigen einer visuellen Darstellung (58) von mindestens einem Abschnitt des Instruments (20) relativ zu den Bilddaten (60) des Objekts (43) auf einem Display.

17. Computerimplementiertes Verfahren (100) nach einem der Ansprüche 13 bis 16, wobei
die visuelle Darstellung (58) ein Symbol umfasst, das zumindest einen Teil eines Kreises (65) oder einer Kugel (66) mit dem Radius (25) des sphärisch geformten Oberflächenabschnitts (26) darstellt.

18. Computerprogrammprodukt, umfassend Anweisungen, die, wenn sie auf mindestens einem Prozessor ausgeführt werden, den mindestens einen Prozessor dazu veranlassen, eines der Verfahren der Ansprüche 1 bis 17 durchzuführen.

19. Vorrichtung (12) zum Bestimmen eines Radius (25) eines sphärisch geformten Oberflächenabschnitts (26) einer Spitze (24) eines Instruments (20), wobei eine Kalibrierungsvorrichtung (34) bereitgestellt ist, die eine ebene Kalibrieroberfläche (45) und eine Kalibrierungsstruktur (38) umfasst, wobei die Kalibrierungsstruktur einen Öffnungswinkel (40) definiert und einen realen oder imaginären ersten Referenzpunkt (44), relativ zu dem der Öffnungswinkel (40) definiert ist, wobei die Kalibrierungsvorrichtung (34) einen ersten Verfolger (36) umfasst, der durch ein Verfolgungssystem (14) verfolgbar ist und in einer ersten vorbestimmten Beziehung (54) relativ zu dem ersten Referenzpunkt (44) und in einer zweiten vorbestimmten Beziehung relativ zu der Kalibrierungsoberfläche (45) angeordnet ist, und wobei das Instrument (20) einen zweiten Verfolger (22) umfasst, der durch das Verfolgungssystem (14) verfolgbar ist, wobei die Vorrichtung ausgebildet ist, um:

eine erste Pose des ersten Verfolgers (36) und eine zweite Pose des zweiten Verfolgers (22) zu bestimmen, während der sphärisch geformte Oberflächenabschnitt (26) an der Kalibrierungsstruktur (38) anliegt; eine Referenzposition (53) des ersten Referenzpunktes (44) relativ zu dem zweiten Verfolger (22) auf der Grundlage der ersten Pose, der zweiten Pose und der ersten vorbestimmten Beziehung (54) zu bestimmen, wenn der erste Verfolger (36) in der ersten Pose und der zweite Verfolger (22) in der zweiten Pose ist; eine dritte Pose des ersten Verfolgers (36) und eine vierte Pose des zweiten Verfolgers (22) zu bestimmen, während der sphärisch geformte Oberflächenabschnitt (26) an der Kalibrierungsoberfläche (45) anliegt; und den Radius (25) des sphärisch geformten Oberflächenabschnitts (26) auf der Grundlage der dritten Pose, der zweiten vorbestimmten Beziehung, der Referenzposition (53), der vierten Pose und des Öffnungswinkels (40) zu bestimmen.

20. Vorrichtung (12) nach Anspruch 19, die ausgebildet ist, um einen der Verfahrensschritte nach einem der Ansprüche 2 bis 6 und 10 bis 17 durchzuführen.

21. Vorrichtung (12) zum Bestimmen eines Radius (25) eines sphärisch geformten Oberflächenabschnitts (26) einer Spitze (24) eines Instruments (20), wobei der sphärisch geformte Oberflächenabschnitt (26) einen Mittelpunkt (27) aufweist, wobei eine Kalibrierungsvorrichtung (34) bereitgestellt ist, die eine Kalibrierungsstruktur (38) umfasst, die einen Öffnungswinkel (40) definiert und die ausgebildet ist, um mit dem sphärisch geformten Oberflächenabschnitt (26) zusammenzuwirken, wobei der sphärisch geformte Oberflächenabschnitt (26) ausgebildet ist, um entlang der Kalibrierungsstruktur (38) in einem Prozess des Führens einer Kippbewegung der Instrumentenspitze (24) um den Mittelpunkt (27) zu gleiten, wobei die Kalibrierungsvorrichtung (34) einen ersten Verfolger (36) umfasst, der durch ein Verfolgungssystem (14) verfolgbar und in einer vorbestimmten Beziehung relativ zu der Kalibrierungsstruktur (38) angeordnet ist, und wobei das Instrument (20) einen zweiten Verfolger (22) umfasst, der durch das Verfolgungssystem (14) verfolgbar ist, wobei die Vorrichtung ausgebildet ist, um:

eine erste Pose des ersten Verfolgers (36) zu bestimmen;
Positionsdaten des zweiten Verfolgers (22) für verschiedene Neigungspositionen des Instruments (20) zu bestimmen, während der sphärisch geformte Oberflächenabschnitt (26) an der Kalibrierungsstruktur (38) anliegt;
eine Position des Mittelpunkts (27) relativ zu dem zweiten Verfolger (22) auf der Grundlage der Positionsdaten zu bestimmen; und
den Radius (25) des sphärisch geformten Oberflächenabschnitts (26) auf der Grundlage der Position des Mittelpunkts (27), der ersten Pose, der vorbestimmten Beziehung und des Öffnungswinkels (40) zu bestimmen.

22. Vorrichtung (12) nach Anspruch 21, die ausgebildet ist, um einen der Verfahrensschritte nach einem der Ansprüche 8 bis 17 durchzuführen.

## Revendications

1. Procédé (100) mis en oeuvre par ordinateur pour déterminer un rayon (25) d'une partie de surface de forme sphérique (26) d'une pointe (24) d'un instrument (20), dans lequel un dispositif d'étalonnage (34) comprend une surface d'étalonnage plate (45) et une structure d'étalonnage (38), la structure d'étalonnage définissant un angle d'ouverture (40) et un premier point de référence réel ou imaginaire (44) par rapport auquel l'angle d'ouverture (40) est défini, le dispositif d'étalonnage (34) comprenant un premier suiveur (36) pouvant être suivi par un système de suivi (14) et disposé dans une première relation prédéterminée (54) par rapport au premier point de référence (44) et dans une seconde relation prédéterminée par rapport à la surface d'étalonnage (45), et dans lequel l'instrument (20) comprend un deuxième suiveur (22) pouvant être suivi par le système de suivi (14), le procédé (100) comprenant les étapes suivantes exécutées par un système informatique (13):

déterminer (202) une première pose du premier suiveur (36) et une deuxième pose du deuxième suiveur (22) alors que la partie de surface de forme sphérique (26) est en butée avec la structure d'étalonnage (38);
déterminer (204) une position de référence (53) du premier point de référence (44) par rapport au deuxième suiveur (22) lorsque le premier suiveur (36) est dans la première pose et que le deuxième suiveur (22) est dans la deuxième pose, en fonction de la première pose, de la deuxième pose et de la première relation prédéterminée (54);
déterminer (206) une troisième pose du premier suiveur (36) et une quatrième pose du deuxième suiveur (22) alors que la partie de surface de forme sphérique (26) est en butée avec la surface d'étalonnage (45); et
déterminer (208) le rayon (25) de la partie de surface de forme sphérique (26) en fonction de la troisième pose, de la deuxième relation prédéterminée, de la position de référence (53), de la quatrième pose et de l'angle d'ouverture (40).

2. Procédé mis en oeuvre par ordinateur (100) selon la revendication 1, dans lequel la détermination du rayon (25) de la partie de surface de forme sphérique (26) comprend

la détermination d'un deuxième point de référence (57) sur la base de la quatrième pose et de la position de référence (53);
la détermination d'une distance de référence (71) définissant une distance entre le deuxième point de référence (57) et la surface d'étalonnage (45) sur la base du deuxième point de référence (57), de la troisième pose et de la deuxième relation prédéterminée; et
la détermination du rayon (25) sur la base de la distance de référence (71) et d'une fonction trigonométrique de l'angle d'ouverture (40).

3. Procédé mis en oeuvre par ordinateur selon la revendication 1 ou 2, dans lequel

la deuxième pose définit une première orientation d'étalonnage du deuxième suiveur (22) et la quatrième pose définit une deuxième orientation d'étalonnage du deuxième suiveur (22), dans lequel un angle de réorientation définit une différence angulaire entre la première orientation d'étalonnage et la deuxième orientation d'étalonnage, dans lequel un angle de surface définit une différence angulaire entre un vecteur normal (49) de la surface d'étalonnage (45) et une bissectrice (42) de l'angle d'ouverture (40), le procédé consistant en outre à déterminer un angle de pivotement (74) en fonction de l'angle de réorientation et de l'angle de surface.

4. Procédé mis en oeuvre par ordinateur (100) selon la revendication 3, dans lequel la détermination (106) du rayon (25) de la partie de surface de forme sphérique (26) est en outre basée sur l'angle de pivotement (74).

5. Procédé mis en oeuvre par ordinateur (100) selon l'une quelconque des revendications précédentes, comprenant en outre

la détermination d'un deuxième point de référence (57) sur la base de la quatrième pose et de la position de référence (53);
la détermination d'un axe de référence (72) passant par le deuxième point de référence (57) et perpendiculaire à la surface d'étalonnage (45); et
la détermination d'une position d'un point central (27) de la partie de surface de forme sphérique (26) par rapport au deuxième suiveur (22) le long de l'axe de référence (72) à une distance du rayon (25) par rapport à la surface de référence dans une direction opposée au dispositif d'étalonnage (34).

6. Procédé mis en oeuvre par ordinateur (100) selon la revendication 5 en combinaison avec l'une des revendications 3 ou 4, comprenant
la rotation de l'axe de référence (72) autour du deuxième point de référence (57) selon l'angle de pivotement (74) avant de déterminer la position du point central (27) le long de l'axe de référence (72).

7. Procédé (100) mis en oeuvre par ordinateur pour déterminer un rayon (25) d'une partie de surface de forme sphérique (26) d'une pointe (24) d'un instrument (20), dans lequel la partie de surface de forme sphérique (26) a un point central (27), dans lequel un dispositif d'étalonnage (34) est fourni qui comprend une structure d'étalonnage (38) définissant un angle d'ouverture (40) et qui est configuré pour coopérer avec la partie de surface de forme sphérique (26), dans lequel la partie de surface de forme sphérique (26) est configurée pour glisser le long de la structure d'étalonnage (38) dans un processus de guidage d'un mouvement d'inclinaison de la pointe de l'instrument (24) autour du point central (27), dans lequel le dispositif d'étalonnage (34) comprend un premier suiveur (36) pouvant être suivi par un système de suivi (14) et disposé dans une relation prédéterminée par rapport à la structure d'étalonnage (38), et dans lequel l'instrument (20) comprend un deuxième suiveur (22) pouvant être suivi par le système de suivi (14), le procédé (100) comprenant les étapes suivantes exécutées par un système informatique (13):

déterminer (102) une première position du premier suiveur (36);
déterminer (104) les données de position du deuxième suiveur (22) pour différentes positions d'inclinaison de l'instrument (20) lorsque la partie de surface de forme sphérique (26) est en butée avec la structure d'étalonnage (38);
déterminer (106) une position du point central (27) par rapport au deuxième suiveur (22) sur la base des données de position; et
déterminer (108) le rayon (25) de la partie de surface de forme sphérique (26) en fonction de la position du point central (27), de la première pose, de la relation prédéterminée et de l'angle d'ouverture (40).

8. Procédé mis en oeuvre par ordinateur (100) selon la revendication 7, comprenant
la détermination du rayon (25) sur la base d'un entr'axe (56) entre la position du point central (27), un point réel ou imaginaire (44) par rapport auquel l'angle d'ouverture (40) est défini et une fonction trigonométrique de l'angle d'ouverture (40).

9. Procédé mis en oeuvre par ordinateur (100) selon la revendication 7 ou 8, dans lequel les données de position comprennent au moins l'un des éléments suivants

- au moins deux poses d'inclinaison différentes du deuxième suiveur (22) pour les différentes positions d'inclinaison, et
- au moins quatre positions différentes du deuxième suiveur (22) pour les différentes positions de d'inclinaison.

**10.** Procédé mise en oeuvre par ordinateur (100) selon l'une quelconque des revendications précédentes, dans lequel la structure d'étalonnage (38) a la forme d'un cône ou d'une pyramide, ou une partie de ces derniers, définissant l'angle d'ouverture (40).

**11.** Procédé mise en oeuvre par ordinateur (100) selon l'une quelconque des revendications précédentes, comprenant la détermination, à partir d'une liste prédéterminée de rayons, d'un sous-ensemble de rayons qui répondent à un critère de similitude par rapport au rayon déterminé.

**12.** Procédé mis en oeuvre par ordinateur selon la revendication 11, comprenant
la génération d'une sortie pour une sélection ou une confirmation de l'utilisateur, la sortie comprenant au moins l'une des informations suivantes: informations sur le sous-ensemble de rayons et informations sur les pointes d'instrument (24) associées au sous-ensemble de rayons.

**13.** Procédé mis en oeuvre par ordinateur selon l'une quelconque des revendications 5, 6 ou 7 à 9, ou selon une revendication qui en découle, comprenant

le suivi (108) d'un troisième suiveur (41) d'un objet (43) pouvant être suivi par le système de suivi (14) pour suivre une pose d'objet de données d'image (60) de l'objet (43) dans un espace virtuel (47);
le suivi (110) du deuxième suiveur (22) pour suivre une position du point central (27) dans l'espace virtuel (47) sur la base de la position du point central (27) par rapport au deuxième suiveur (22); et
la génération (112) de premières instructions d'affichage pour afficher dans l'espace virtuel (47), sur la base de la pose de l'objet des données d'image (60) de l'objet (43) dans l'espace virtuel (47), de la position du point central (27) dans l'espace virtuel (47) et du rayon (25), une représentation visuelle (58) indiquant le rayon (25) par rapport aux données d'image (60) de l'objet (43).

**14.** Procédé mis en oeuvre par ordinateur (100) selon la revendication 13, comprenant
la génération d'instructions d'affichage de région pour afficher une région non cible (63) dans les données d'image (60) pour laquelle le contact avec l'instrument (20) doit être évité.

**15.** Procédé mis en oeuvre par ordinateur (100) selon la revendication 13 ou 14, comprenant
la génération d'»instructions d'affichage de la progression pour afficher la progression (67) de la manipulation de l'objet en fonction de la trajectoire de la pointe de l'instrument (24).

**16.** Procédé mis en oeuvre par ordinateur (100) selon l'une quelconque des revendications 13 ou 15, comprenant
l'affichage, sur un écran, d'une représentation visuelle (58) d'au moins une partie de l'instrument (20) par rapport aux données d'image (60) de l'objet (43).

**17.** Procédé mis en oeuvre par ordinateur (100) selon l'une quelconque des revendications 13 à 16, dans lequel la représentation visuelle (58) comprend une icône représentant au moins une partie d'un cercle (65) ou d'une sphère (66) avec le rayon (25) de la partie de surface de forme sphérique (26).

**18.** Produit-programme informatique, comprenant des instructions qui, lorsqu'elles sont exécutées sur au moins un processeur, amènent l'au moins un processeur à mettre en oeuvre l'un des procédés des revendications 1 à 17.

**19.** Dispositif (12) pour déterminer un rayon (25) d'une partie de surface de forme sphérique (26) d'une pointe (24) d'un instrument (20), dans lequel un dispositif d'étalonnage (34) est fourni qui comprend une surface d'étalonnage plate (45) et une structure d'étalonnage (38), dans laquelle la structure d'étalonnage (38) définit un angle d'ouverture (40) et un premier point de référence réel ou imaginaire (44) par rapport auquel l'angle d'ouverture (40) est défini, dans lequel le dispositif d'étalonnage (34) comprend un premier suiveur (36) pouvant être suivi par un système de suivi (14) et disposé dans une première relation prédéterminée (54) par rapport au premier point de référence (44) et dans une seconde relation prédéterminée par rapport à la surface d'étalonnage (45), et dans lequel l'instrument (20) comprend un deuxième suiveur (22) pouvant être suivi par le système de suivi (14), le dispositif étant configuré pour

déterminer une première position du premier suiveur (36) et une seconde position du deuxième suiveur (22) alors que la partie de surface de forme sphérique (26) est en contact avec la structure d'étalonnage (38) ;
déterminer une position de référence (53) du premier point de référence (44) par rapport au deuxième suiveur (22) lorsque le premier suiveur (36) est dans la première pose et que le deuxième suiveur (22) est dans la deuxième pose, sur la base de la première pose, de la deuxième pose et de la première relation prédéterminée (54);

déterminer une troisième pose du premier suiveur (36) et une quatrième pose du deuxième suiveur (22) alors que la partie de surface de forme sphérique (26) est en butée avec la surface d'étalonnage (45); et

déterminer le rayon (25) de la partie de surface de forme sphérique (26) en fonction de la troisième pose, de la deuxième relation prédéterminée, de la position de référence (53), de la quatrième pose et de l'angle d'ouverture (40).

20. Dispositif (12) selon la revendication 19, configuré pour exécuter l'une quelconque des étapes de procédé selon les revendications 2 à 6 et 10 à 17.

21. Dispositif (12) pour déterminer un rayon (25) d'une partie de surface de forme sphérique (26) d'une pointe (24) d'un instrument (20), dans lequel la partie de surface de forme sphérique (26) a un point central (27), dans lequel un dispositif d'étalonnage (34) comprend une structure d'étalonnage (38) définissant un angle d'ouverture (40) et qui est configuré pour coopérer avec la partie de surface de forme sphérique (26), la partie de surface de forme sphérique (26) étant configurée pour glisser le long de la structure d'étalonnage (38) dans un processus de guidage d'un mouvement d'inclinaison de la pointe d'instrument (24) autour du point central (27), le dispositif d'étalonnage (34) comprenant un premier suiveur (36) pouvant être suivi par un système de suivi (14) et se trouvant dans une relation prédéterminée par rapport à la structure d'étalonnage (38), et dans lequel l'instrument (20) comprend un deuxième suiveur (22) pouvant être suivi par le système de suivi (14), le dispositif étant configuré pour

déterminer une première pose du premier suiveur (36);

déterminer des données de position du deuxième suiveur (22) pour différentes positions d'inclinaison de l'instrument (20) lorsque la partie de surface de forme sphérique (26) est en butée avec la structure d'étalonnage (38);

déterminer une position du point central (27) par rapport au deuxième suiveur (22) sur la base des données de position; et

déterminer le rayon (25) de la partie de surface de forme sphérique (26) en fonction de la position du point central (27), de la première pose, de la relation prédéterminée et de l'angle d'ouverture (40).

22. Dispositif (12) selon la revendication 21, configuré pour effectuer l'une quelconque des étapes du procédé selon les revendications 8 à 17.

Fig. 1A

Fig. 1B

Fig. 1C

Fig. 1D

Fig. 2B

Fig. 2A

Fig. 3

EP 3 984 485 B1

Fig. 4A          Fig. 4B          Fig. 4C

Fig. 5B

Fig. 5A

Fig. 6C

Fig. 6D

Fig. 6A

Fig. 6B

Fig. 7A

Fig. 7B

100

| | |
|---|---|
| 102 | Determine a first pose of a first tracker |
| 104 | Determine positional data of a second tracker for different tilting positions of an instrument while a spherically shaped surface portion is in abutment with a calibration structure |
| 106 | Determine a position of a centre point relative to the second tracker based on the positional data |
| 108 | Determine a radius of the spherically shaped surface portion based on the position of the centre point, the first pose, a predetermined relationship, and an opening angle |

Fig. 8

EP 3 984 485 B1

Fig. 9B

Fig. 9A

Fig. 10

Fig. 11

EP 3 984 485 B1

Fig. 12B

Fig. 12A

200

| 202 | Determine a first pose of a first tracker and a second pose of a second tracker while a spherically shaped surface portion is in abutment with a calibration structure |
| --- | --- |
| 204 | Determine a reference position of a first reference point relative to the second tracker when the first tracker is in the first pose and the second tracker is in the second pose based on the first pose, the second pose, and a first predetermined relationship |
| 206 | Determine a third pose of the first tracker and a fourth pose of the second tracker while the spherically shaped surface portion is in abutment with a calibration surface |
| 208 | Determine a radius of the spherically shaped surface portion based on the third pose, a second predetermined relationship, the reference position, the fourth pose, and an opening angle |

Fig. 13

EP 3 984 485 B1

Fig. 14A

Fig. 14B

EP 3 984 485 B1

Fig. 15A

Fig. 15B

EP 3 984 485 B1

Fig. 16B

Fig. 16A

Fig. 17

Fig. 18A

Fig. 18B

Fig. 18C

Fig. 18D

Fig. 18E

Fig. 18F

EP 3 984 485 B1

49

Fig. 19

EP 3 984 485 B1

Fig. 20A

Fig. 20B

EP 3 984 485 B1

Fig. 21

Fig. 22A

Fig. 22B

Fig. 22C

EP 3 984 485 B1

**Fig. 23A**

- 3.5 mm
- 3.6 mm
- 3.7 mm
- 3.8 mm
- 3.9 mm

78

measurement:
3.72 mm

80

**Fig. 23B**

- 3.3 mm
- 3.4 mm
- 3.5 mm
- 3.6 mm
- 3.7 mm
- 3.8 mm
- 3.9 mm
- 4.0 mm
- 4.1 mm
- 4.2 mm

78

81

measurement:
3.72 mm

80

**Fig. 23C**

81

78

| radius | name | material | select |
|--------|--------|------------|--------|
| • 3.5 mm | name 1 | material 1 | ◯ |
| • 3.6 mm | name 2 | material 2 | ◯ |
| • 3.7 mm | name 3 | material 1 | ◯ |
| • 3.8 mm | name 4 | material 3 | ◯ |
| • 3.9 mm | name 5 | material 2 | ◯ |

82

**Fig. 23D**

81

78

| radius | name | tip shape | select |
|--------|--------|-----------|--------|
| • 3.6 mm | name 1 | | ◯ |
| • 3.6 mm | name 2 | | ◯ |
| • 3.7 mm | name 3 | | ◯ |
| • 3.7 mm | name 4 | | ◯ |
| • 3.8 mm | name 5 | | ◯ |

82

Fig. 24C

Fig. 24B

Fig. 24A

**EP 3 984 485 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020405400 A1
- US 2020046454 A1